(19) Europäisches Patentamt
European Patent Office
Office européen des brevets

(11) EP 3 983 563 B1

(12) **EUROPEAN PATENT SPECIFICATION**

(45) Date of publication and mention of the grant of the patent:
**25.03.2026 Bulletin 2026/13**

(21) Application number: **20735800.3**

(22) Date of filing: **16.06.2020**

(51) International Patent Classification (IPC):
***C12Q 1/6883*** *(2018.01)* ***A61B 5/00*** *(2006.01)*
***A61P 43/00*** *(2006.01)*

(52) Cooperative Patent Classification (CPC):
**C12Q 1/6883; A61B 5/4088; A61P 43/00;**
C12Q 2600/106

(86) International application number:
**PCT/US2020/037878**

(87) International publication number:
**WO 2020/257159 (24.12.2020 Gazette 2020/52)**

(54) **METHODS FOR TREATING NEURODEGENERATIVE DISORDERS**

VERFAHREN ZUR BEHANDLUNG NEURODEGENERATIVER ERKRANKUNGEN

PROCÉDÉS DE TRAITEMENT DE TROUBLES NEURODÉGÉNÉRATIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2019 US 201962862400 P**
**05.12.2019 US 201962944179 P**

(43) Date of publication of application:
**20.04.2022 Bulletin 2022/16**

(73) Proprietor: **Alzheon, Inc.**
**Framingham, MA 01701 (US)**

(72) Inventors:
- **ABUSHAKRA, Susan**
  **Framingham, MA 01701 (US)**
- **HEY, John**
  **Framingham, MA 01701 (US)**
- **POWER, Aidan**
  **Framingham, MA 01701 (US)**
- **TOLAR, Martin**
  **Framingham, MA 01701 (US)**

(74) Representative: **Potter Clarkson**
**Chapel Quarter**
**Mount Street**
**Nottingham NG1 6HQ (GB)**

(56) References cited:
**WO-A1-2018/157014 WO-A2-2015/143447**

- **S. ABUSHAKRA ET AL: "Clinical Benefits of Tramiprosate in Alzheimer's Disease Are Associated with Higher Number of APOE4 Alleles: The "APOE4 Gene-Dose Effect"", J PREV ALZ DIS, 24 October 2016 (2016-10-24), pages 219 - 228, XP055569473, Retrieved from the Internet <URL:http://dx.doi.org/10.14283> DOI: 10.14283/jpad.2016.115**
- **CORDER E H ET AL: "GENE DOSE OF APOLIPOPROTEIN E TYPE 4 ALLELE AND THE RISK OF ALZHEIMER'S DISEASE IN LATE ONSET FAMILIES", SCIENCE, AMERICAN ASSOCIATION FOR THE ADVANCEMENT OF SCIENCE, US, vol. 261, 13 August 1993 (1993-08-13), pages 921 - 923, XP000619582, ISSN: 0036-8075, DOI: 10.1126/SCIENCE.8346443**
- **GAUTHIER S ET AL: "Effect of tramiprosate in patients with mild-to-moderate alzheimer's disease: Exploratory analyses of the MRI sub-group of the alphase study", JOURNAL OF NUTRITION, HEALTH AND AGING, SPRINGER PARIS, PARIS, vol. 13, no. 6, 4 July 2009 (2009-07-04), pages 550 - 557, XP036030410, ISSN: 1279-7707, [retrieved on 20090704], DOI: 10.1007/S12603-009-0106-X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 983 563 B1

- **JACK CLIFFORD R ET AL: "Defining imaging biomarker cut points for brain aging and Alzheimer's disease", ALZHEIMER'S & DEMENTIA: THE JOURNAL OF THE ALZHEIMER'SASSOCIATION, ELSEVIER, NEW YORK, NY, US, vol. 13, no. 3, 30 September 2016 (2016-09-30), pages 205 - 216, XP029941903, ISSN: 1552-5260, DOI: 10.1016/ J.JALZ.2016.08.005**

Remarks:

The file contains technical information submitted after the application was filed and not included in this specification

## Description

## RELATED APPLICATIONS

## BACKGROUND

Individuals with late onset Alzheimer's disease (AD) comprise a heterogeneous population with variable trajectories of disease progression. This variability, both in the rates of clinical decline and biomarker changes, likely played a significant role in the failure of numerous amyloid immunotherapies, despite the requirement for positive markers of amyloid pathology in recent clinical trials. Although the diagnosis of AD requires the presence of amyloid and tau pathology, this variability suggests the presence of underlying biological differences among the clinical phenotypes of AD. See Veitch DP, Weiner MW, Aisen PS, Beckett LA, Cairns NJ, Green RC, et al. Understanding disease progression and improving Alzheimer's disease clinical trials: Recent highlights from the Alzheimer's Disease Neuroimaging Initiative. Alzheimers Dement 2019;15:106-52. Identification of AD subtypes with more homogenous clinical course would be a major advance for successful drug development.

A known genetic variant with a robust effect on AD biology and disease progression is the apolipoprotein ε allele (*APOE*), which occurs as *APOE 2, 3*, or *4*. The *APOE4* allele is a major genetic risk factor for late onset AD, with a gene-dose effect on the risk of dementia. See Corder EH, Saunders AM, Strittmatter WJ, Schmechel DE, Gaskell PC, Small GW, et al. Gene dose of apolipoprotein E type 4 allele and the risk of Alzheimer's disease in late onset families. Science 1993;261:921-3 and Roses AD. Apolipoprotein E alleles as risk factors in Alzheimer's disease. Annu Rev Med 1996;47:387-400. Based on a large body of evidence, described below, AD patients with two ε4 alleles (APOE4/4 homozygotes) exhibit a unique biological and clinical profile that makes them an attractive population for drug trials.

The *APOE4* allele is known to increase the risk of AD and accelerate its onset, while *APOE3* is considered the neutral "wild-type", and *APOE2* appears protective. The risk of AD increases approximately 4-fold in APOE4 heterozygotes with one ε4 allele (most commonly APOE3/4), and approximately 14-fold in APOE4/4 homozygotes. This effect of APOE4 is related to increased neuronal production of beta amyloid (Aβ) and its decreased clearance from the interstitial space, leading to a high prevalence of amyloid pathology in elderly APOE4 carriers (see e.g., Reiman EM et al., PNAS 2009;106:6820-25 and Jansen et al., JAMA 2015;313:1924-38). Among APOE4/4 AD patients, approximately 95% show fibrillar amyloid deposition (see e.g., Ossenkoppele R et al. JAMA 2015;313:1939-49 and Degenhardt EK, et al., Psychosomatics 2016;57:208-216). Importantly, APOE4/4 AD patients have approximately 3-fold higher brain levels of soluble neurotoxic Aβ oligomers than non-carriers (see e.g., . See Hashimoto T et al., J Neurosci 2012;32:15181-92; Tai LM, Bilousova T et al., J Biol Chem 2013;288(8):5914-26; Viola KL et al., Acta Neuropathol 2015;129:183-206; and Hong W et al., Acta Neuropathol 2018;136:19-40). APOE4/4 AD patients also show high levels of cerebrospinal fluid (CSF) p-$tau_{181}$ (see e.g, Apostolova LG, et al., Neurobiol Aging 2010;31:1284-1303), and early onset of hippocampus-dependent cognitive deficits (see e.g., Reiman EM et al., PNAS 2001;98:3334-39; Scarmeas N et al., Am J Geriatr Psychiatry 2004;12:596-605; and Caselli RJ et al., N Engl J Med 2009;361:255-63).

APOE4/4 homozygotes therefore comprise an AD population with distinct biological features, enriched with soluble Aβ oligomers, which correlate with the onset and severity of clinical symptoms (see e.g., Esparza TJ et al., Ann Neurol 2013;73:104-19). For agents that inhibit formation of Aβ oligomers, such as tramiprosate/ALZ-801, APOE4/4 homozygotes constitute an optimal population to evaluate clinical efficacy. See e.g., Kocis P et al., CNS Drugs 2017;31:495-509; Hey JA, et al., CNS Drugs 2018;32:849-861; and Abushakra S et al., J Prev Alz Dis 2016; 3(4):219-28.

Tramiprosate, 3-amino-1-propanesulfonic acid (3APS) is an oral amyloid anti-aggregation agent which inhibits formation of amyloid beta oligomers and reduces oligomer related neurotoxicity in the brain. The tramiprosate Phase 3 trials in mild-to-moderate AD showed promising dose-dependent effects on MRI imaging, namely slowing the reduction of brain hippocampal volume (hippocampus atrophy), see e.g., Gauthier, S. et al., J Nutr Health Aging 13, 550-557 (2009). Tramiprosate also showed positive cognitive efficacy in the subset of APOE4 carriers, especially APOE4/4 homozygotes. See e.g., S. Abushakra et al., J Prev Alz Dis 2016; 3(4):219-28.

ALZ-801 is in clinical development as an oral, small molecule inhibitor of beta amyloid (Aβ) oligomer formation for the treatment of Alzheimer's disease (AD). ALZ-801 is a valine conjugate of tramiprosate with improved pharmacokinetic properties and gastrointestinal tolerability (see e.g., Hey et al., Clin Pharmacokinetics 2018; 315-333). Tramiprosate, the active moiety of ALZ-801, inhibits the formation of Aβ oligomers *in vitro* (see e.g., Kocis et al., CNS Drugs 2017; 31:495-509). Oral tramiprosate was previously evaluated in two Phase 3 studies, which included 2,015 patients with mild to moderate AD, treated with 100 mg BID of tramiprosate, 150 mg BID of tramiprosate, or placebo over 78 weeks. Safety data from these Phase 3 trials and the safety extension study, suggest a favorable safety profile with tramiprosate exposures of up to 2.5 years. See e.g., Abushakra et al., J Prev Alzheimers Dis 2017; 4:149-56. In a subgroup analysis of subjects with two copies of the ε4 allele of apolipoprotein E (APOE4/4 homozygotes), there was a positive and clinically meaningful benefit on cognition.

## SUMMARY

The ability to identify and treat subjects who are prone to developing Alzheimer's disease before they exhibit the cognitive symptoms of Alzheimer's disease is crucial because once symptoms appear, it may be too late to reverse such symptoms or to prevent further cognitive decline (see e.g., D Mehta et al., Expert Opin Investig Drugs. 2017, 26(6): 735-739). Here, the present inventors discovered that decreases in hippocampal volume and cortical thickness in otherwise normal human subjects (e.g., subjects who demonstrate little or no cognitive decline) is a strong predictor of Alzheimer's disease (AD), and is therefore an early biomarker for the treatment of AD. Accordingly, provided herein are methods for using hippocampal volume (HV) atrophy and/or cortical thickness (CT) as markers to identify human subjects who are likely to be responsive to treatment with AD drugs such as tramiprosate, a pharmaceutically acceptable salt of tramiprosate, a tramiprosate prodrug, a tramiprosate derivative, or an active tramiprosate metabolite.

Volumetric MRI Imaging and clinical data from the longitudinal Alzheimer's Disease Neuroimaging Initiative study (ADNI-1) was analyzed. In this dataset, we evaluated the rates of hippocampus atrophy and cortical thickness loss in APOE4/4 versus APOE3/3 subjects who participated in the tramiprosate trials, and their correlation with cognitive decline on 2 standard cognitive tests (ADAS-cog and MMSE). From this study population, we discovered higher rates of hippocampus volume (HV) atrophy in APOE4/4 subjects with mild cognitive impairment (MCI) and mild Alzheimer's disease (AD), compared to APOE3/3 subjects. See e.g., **Table 4,** where in APOE4/4 subjects with MCI, the rate of HV loss was 4.41 $cm^3$ at 12 months, and 9.73 $cm^3$ at 24 months, compared to 2.76 $cm^3$ at 12 months, and 6.17 $cm^3$ at 24 months in APOE3/3 subjects. Similarly, in APOE4/4 subjects with mild AD, the rate of HV loss was 7.44 $cm^3$ at 12 months, and 16.10 $cm^3$ at 24 months, compared to rates of 5.43 mm and 10.74 $cm^3$ in APOE3/3 subjects, respectively.

We also identified that the APOE4/4 MCI and mild AD subjects had significantly higher rates of cortical thickness loss at 12 and 24 months than APOE3/3 subjects. See e.g., **Table 4,** where in APOE4/4 subjects with MCI, the rate of cortical thickness loss was 0.09 mm (SD 0.03) at 12 months, and 0.17 mm (SD 0.06) at 24 months, compared to 0.05 mm (SD 0.04) at 12 months, and 0.09 mm (SD 0.07) at 24 months in APOE3/3 subjects. Similarly, in APOE4/4 subjects with mild AD, the rate of cortical thickness loss was 0.12 mm (SD 0.06) at 12 months, and 0.22 mm (SD 0.08) at 24 months, compared to rates of 0.09 mm (SD 0.04) and 0.17 mm (SD 0.08) in APOE3/3 subjects, respectively.

Interestingly, it was also discovered that in APOE4/4 subjects with MCI, atrophy rates of HV and cortical thickness over 24 months were significantly correlated with worsening of the cognitive scores on ADAS-cog and MMSE. For example, the correlations of HV atrophy to ADAS-cog and MMSE were $r = -0.55$, $P = 0.002$ and $r = 0.39$, $P = 0.037$, respectively. The correlations of cortical thickness loss to ADAS-cog and MMSE were also significant ($r = -0.59$, $P < 0.001$) and ($r = 0.38$, $P = 0.041$), respectively. These correlations at the early stages of AD establish the use of HV and/or cortical thickness as early markers of disease progression, indicating that such subjects are at risk of disease progression or clinical worsening. As such, these data support the use of HV, cortical thickness, or both, as early indicators of when treatment with AD drugs should be established prior to the appearance of cognitive deficits, e.g., as a preventive treatment at the presymptomatic stage. The data also support the utility of HV, cortical thickness, or both to assess the efficacy of AD drugs on slowing disease progression.

Taken together, this data establishes a strong correlation between cognitive decline and the rates of HV atrophy and CT loss in APOE4/4 human subjects with mild cognitive impairment (MCI). Accordingly, in one aspect, the present methods are particularly useful for identifying APOE4/4 human subjects who are at risk for cognitive decline, and likely to need early treatment at the asymptomatic stage (and treating them).

Thus, treating APOE4/4 subjects with HV or cortical thickness values below certain threshold levels with agents that inhibit formation of Aβ oligomers should protect asymptomatic AD subjects from cognitive decline. This type of treatment in APOE4/4 subjects with reduced HV or cortical thickness should also reduce the likelihood of worsening cognitive decline in subjects having mild AD. Also provided herein, therefore are methods for treating APOE4/4 subjects who are asymptomatic (e.g., with subtle or no cognitive deficits) for AD and who have at least one of a HV or cortical thickness below a certain threshold value. Further provided are methods for treating APOE4/4 subjects who have MCI or mild AD and who have at least one of a HV or cortical thickness below a certain threshold value.

Other subjects that may benefit from the disclosed methods are contemplated and include e.g., those with genotype other than APOE4/4 who have mild AD, MCI, or are presymptomatic, and are determined to be at risk of cognitive decline based on their HV and/or CT measures, particularly in subjects who have also been determined to have one APOE4 allele and/or who have been determined to be positive for amyloid deposits in the brain.

## BRIEF DESCRIPTION OF THE FIGURES

**FIG. 1** shows the mean annualized hippocampus atrophy rates in APOE4/4 homozygotes from tramiprosate and ADNI-1 vMRI datasets.

## DETAILED DESCRIPTION

### A. Definitions

The term "adult human subject", "adult subject", "adult human patient", and "adult patient" are used interchangeably and refer to a human subject who is 18 years of age or older. In some aspects, the human subject is 50 or 55 years of age or older. In other aspect, the human subject is 85 years of age or less. In other aspects, the subject is human age 65-85 years old.

"Tramiprosate" (homotaurine, 3-amino-1-propanesulfonic acid (3-APS), or Alzhemed™) is an oral amyloid anti-aggregation agent which inhibits formation of amyloid beta oligomers and reduces oligomer-related neurotoxicity in the brain. Tramiprosate was investigated in Phase 3 clinical trials for treating subjects with mild-to-moderate AD. See e.g., Gauthier, S. et al. Effect of tramiprosate in patients with mild-to-moderate Alzheimer's disease: exploratory analyses of the MRI sub-group of the Alphase study. J Nutr Health Aging 13, 550-557 (2009); Saumier, D., Duong, A., Haine, D., Garceau, D. & Sampalis, J. Domain-specific cognitive effects of tramiprosate in patients with mild to moderate Alzheimer's disease: ADAS-cog subscale results from the Alphase Study. J Nutr Health Aging 13, 808-812 (2009); and Aisen, P. S. et al. Tramiprosate in mild-to-moderate Alzheimer's disease - a randomized, double-blind, placebo-controlled, multi-centre study (the Alphase Study). Arch Med Sci 7, 102-111 (2011). Tramiprosate has the following chemical structure:

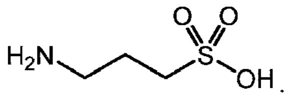

A "tramiprosate prodrug" or "prodrug of tramiprosate" refers to a chemical compound that, after administration to a subject, is metabolized into tramiprosate. Such prodrugs include, but art not limited to, those having the formula:

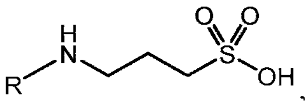

and pharmaceutically acceptable salts, wherein R is $(AA^1)_q(AA^2)_t$-H; $AA^1$ and $AA^2$ are each independently selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), β-alanine (β-ALA), and γ-aminobutyric acid (GABA); q is 1; and t is 0 or 1. Additional prodrugs can be found in WO 2015/143447. In certain aspects, the tramiprosate prodrug is ALZ-801 (valyl-3-amino-1-propanesulfonic acid), or a pharmaceutically acceptable salt thereof. ALZ-801 is in clinical development as an oral, small molecule inhibitor of beta amyloid (Aβ) oligomer formation for the treatment of Alzheimer's disease (AD). ALZ-801 (valyl-3-amino-1-propanesulfonic acid) is an orally bioavailable valine prodrug of tramiprosate that has improved pharmacokinetics and oral tolerability over tramiprosate. Phase 1b clinical pharmacology studies with ALZ-801 show bioequivalence to plasma exposures to tramiprosate from the Phase 3 studies. See e.g., WO 2017/044840. The structure of ALZ-801 is shown below.

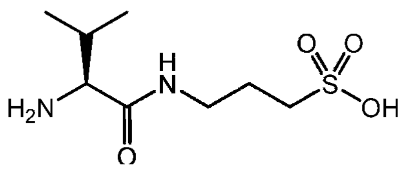

An "active tramiprosate metabolite" refers to a metabolized form of tramiprosate which continues to produce effects in the body. Active tramiprosate metabolites include the compound having the formula

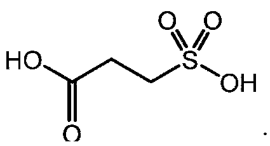

and pharmaceutically acceptable salts thereof. See e.g., US 62/713056.

A "prodrug of an active tramiprosate metabolite" refers to a chemical compound that, after administration to a subject, is metabolized into an active tramiprosate metabolite. Examples of such prodrugs include e.g., those having the

formula

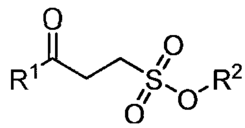

and pharmaceutically acceptable salts thereof, wherein the definitions of $R^1$ and $R^2$ each are as described in US 62/713,056.

As used herein, the term "asymptomatic" when used in connection with AD, such as "asymptomatic for Alzheimer's disease" refers to subjects who do not present cognitive symptoms, do not show cognitive decline, or who have only very subtle cognitive deficits which are detectable by thorough neuropsychological testing. Cognitive assessments can be performed based on methods known in the art and include e.g., the Mini-Mental State Exam (MMSE) and Repeatable Battery for the Assessment of Neuropsychological Status (RBANS) (Duff et al., Archives of Clinical Neuropsychology 23 (2008) 603-612 and Clin Neuropsychol. 2003 Aug;17(3):351-366), and the Alzheimer's Disease Assessment Scale-cognitive subscale (ADAS-cog) (Graham et al., 2004, Alzheimer Dis Assoc Disord 18, 236-240). In one aspect, the subject is "asymptomatic" for Alzheimer's disease if their cognitive symptoms, cognitive decline, and cognitive deficits are age appropriate, i.e., some level of cognitive impairment is excepted as age progresses, which is not connected to AD. In another aspect, the term "asymptomatic" also refers to subjects (e.g., age-appropriate subjects) who do not present with any complaints or concerns about loss of memory. In another aspect, a subject is defined as being asymptomatic if his/her MMSE score is 28 or greater. In other aspects, a subject is defined as being asymptomatic if his/her MMSE score is 29 or greater. In one aspect, the subject is asymptomatic if his/her MMSE score is 30. In still other aspects, a subject is defined as being asymptomatic if they have an APOE2/4 genotype and an ADAS-cog score of 17 or greater. In still other aspects, a subject is defined as being asymptomatic if they have an APOE3/4 or APOE4/4 genotype and an ADAS-cog score of 20 or greater. In another aspect, a subject is defined as being asymptomatic if his/her CDR-global score is less than 0.5. Combinations and alternatives of the above of MMSE scores, ADAS-cog scores and CDR-global score used to define "asymptomatic" subjects are also included.

Subjects with mild AD refers to those subjects who have an MMSE score of 20-26 and includes subjects having MMSE scores of 20, 21, 22, 23, 24, 25, and 26.

Subjects with mild cognitive impairment (MCI) refers to those subjects who have an MMSE score of 24 or greater and includes subjects having MMSE scores of 24, 25, 26, 27, 28, 29 and 30. In one aspect, subjects with MCI refers to those subjects who have an MMSE score of greater than 26.

Subjects with early AD are characterized as those who have mild AD or MCI and includes e.g., those subjects having MMSE score of 20 or greater. In aspect, subjects with early AD are characterized as those with MMSE score of 20 to 30 (e.g., 20, 21, 22, 23, 24, 25, 26, 27, 28, 29, or 30).

The term "threshold level" as used herein refers to a level below which a recited property (e.g., hippocampal volume or cortical thickness) is considered abnormally low. Subjects having one or more properties whose values are below the threshold level are categorized into a first group that are selected for treatment. Subjects having all properties at or above the threshold level are categorized into a second group that is not selected for treatment. In the present invention, subjects having hippocampal volume and/or cortical thickness below their respective threshold levels are considered at risk of developing one or more symptoms of cognitive decline or AD. In some aspects, the threshold level can be determined by 1) determining the mean value of the desired property or generating a standard curve for a desired property, such as hippocampal volume or cortical thickness, from a random population of at least 25, at least 50, at least 100, at least 500 normal adult human subjects; then 2) calculating 1 standard deviation unit (SDv) below the mean value, and 3) calculating the threshold level as the value corresponding to at least 10% below the SDv. In some aspects, the value is 10% below the SDv. In other aspects, the value is at least 11% below, at least 12% below, at least 13% below, at least 14% below, at least 15% below, or at least 20% below the SDv. In other aspects, the value is 11% below, 12% below, 13% below, 14% below, 15% below, or 20% below the SDv. In this manner, the subject who is a candidate for selection need only have a value for the desired property measured at the time of selection and that value is then compared to the threshold to determine if the subject is selected. In some aspects, the threshold level determined above may be age-adjusted to align with the age of the subject who is being evaluated for selection. This may be achieved by generating a first standard curve in a normal population for the value of the desired property vs age; and then generating a second standard curve, which is 1 SDv plus a certain % below the first standard curve, that represents the threshold value at each age. In some aspects, the normal population used to obtain the threshold value have the same ApoE genotype and different threshold values for different APOE4 genotypes may be generated. In some aspects, all of the members of the normal population used to obtain the threshold value are $APOE4^+$. In some aspects, all of the members of the normal population used to obtain the threshold value are APOE4 homozygous. In alternate aspects, the threshold level is based upon a change from a baseline measure of hippocampal volume or cortical thickness obtained from the same subject 6-36 months prior to selection

(longitudinal change over time). In some aspects, the threshold level is based upon a baseline measure of hippocampal volume or cortical thickness obtained from the same subject 12-36 months prior to selection. Other specific threshold values are described herein.

An "age adjusted" value such as age-adjusted hippocampal volume or cortical thickness refers to a corrected value based on the average age of the normal population tested and a value determined from a standard population. In one aspect, "age adjusted" means the threshold value determined for subjects +/- 5 years of age of the subject at the time of the determination of hippocampal volume or cortical thickness for selection.

Amyloid deposits can be determined by methods known in the art and include e.g., PET imaging or cerebral spinal fluid biomarkers. See e.g., Nabers et al., EMBO Molecular Medicine e8763/2018 and Forlenza et al., Alzheimer's and Dementia: Diagnosis, Assessment and Disease Monitoring 1 (2015) 455-463.

An "APOE4 positive" or "APOE4$^+$" subject refers to a human subject who has at least one APOE4 allele.

An "APOE4 homozygous" subject refers to a human subject who has two APOE4 alleles.

As used herein, the term "treat", "treating" or "treatment" means reversing, alleviating, or inhibiting the progress of a neurodegenerative disease such as AD and cognitive decline, or one or more symptoms associated therewith.

The term "pharmaceutically acceptable salt" is a salt of a basic group (e.g., an amino group) or of an acidic group (e.g., a sulfonic acid) on the compounds described herein. Illustrative salts of a basic group include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucoronate, saccharate, formate, benzoate, glutamate, methanesulfonate, ethanesulfonate, benzenesulfonate, p-toluenesulfonate, camphorsulfonate, and pamoate (*i.e.*, 1,1 '-methylene-bis-(2-hydroxy-3-naphthoate)) salts. Illustrative salts of an acidic group include, but are not limited, to lithium, sodium, potassium, calcium, magnesium, aluminum, chromium, iron, copper, zinc, cadmium, ammonium, guanidinium, pyridinium, and organic ammonium salts.

"Pharmaceutically acceptable" refers to drugs, medicaments, inert ingredients etc., which the term describes, suitable for use in contact with the tissues of humans and lower animals without undue toxicity, incompatibility, instability, irritation, allergic response, and the like, commensurate with a reasonable benefit/risk ratio. In one aspect, pharmaceutically acceptable refers to a compound or composition that is approved or approvable by a regulatory agency of the Federal or state government or listed in the U.S. Pharmacopoeia or other generally recognized pharmacopoeia for use in animals and more particularly in humans.

Methods of administration can use an amount and a route of administration effective for treating or lessening the severity of a disease described herein. The exact amount required will vary from subject to subject, depending on the species, age, and general condition of the subject, the severity of the infection, the particular agent, its mode of administration, and the like. Provided compounds are preferably formulated in unit dosage form for ease of administration and uniformity of dosage. For example, provided compounds may be formulated such that a dosage of between 0.01 - 100 mg/kg body weight/day of the compound can be administered to a patient receiving these compositions. It will be understood, however, that the total daily usage of the compounds and compositions of the present disclosure will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts.

It should also be understood that a specific dosage and treatment regimen for any particular patient will depend upon a variety of factors, including age, body weight, general health, sex, diet, time of administration, rate of excretion, drug combination, the judgment of the treating physician, and the severity of the particular disease being treated.

### B. Embodiments

In a first embodiment, provided herein is a method of selecting and treating an adult human subject comprising the steps of: a) selecting an adult human subject who is determined to:

i) have at least one APOE4 allele; ii) be asymptomatic for Alzheimer's disease; and iii) have at least one of: (1) a hippocampal volume (HV) below a threshold level or (2) a cortical thickness below a threshold level; and b) administering to the selected human subject a therapeutically effective amount of tramiprosate or a pharmaceutically acceptable salt of tramiprosate, a tramiprosate prodrug, a tramiprosate derivative, or an active tramiprosate metabolite.

In a second embodiment, subjects described herein (e.g., as in the first embodiment) are selected on the basis of

having a hippocampal volume below a threshold level.

In a third embodiment, subjects described herein (e.g., as in the first or second embodiment) are selected on the basis of having a hippocampal volume below a threshold level, based upon rate of change in an hippocampal volume obtained from the same subject 6-36 months prior to selection.

In a fourth embodiment, subjects described herein (e.g., as in the first or second embodiment) are selected on the basis of having a hippocampal volume below a threshold level, wherein the threshold level is based upon the average age-adjusted hippocampal volume determined from a population of normal humans.

In a fifth embodiment, subjects described herein (e.g., as in the first embodiment) are selected on the basis of having a cortical thickness below a threshold level.

In a sixth embodiment, subjects described herein (e.g., as in the first or fifth embodiment) are selected on the basis of having a cortical thickness below a threshold level based upon rate of change from a baseline measure of cortical thickness obtained from the same subject 12-36 months prior to selection.

In a seventh embodiment, subjects described herein (e.g., as in the in the first or fifth embodiment) are selected on the basis of having a cortical thickness based upon the average age adjusted cortical thickness determined in a population of normal humans.

In an eighth embodiment, subjects described herein (e.g., as in the first to seventh embodiments) are selected on the basis of having both a cortical thickness below a threshold level and a hippocampal volume below a threshold level.

In a ninth embodiment, subjects described herein (e.g., as in the first to eighth embodiments) are only selected if the subject is determined to have two APOE4 alleles.

In a tenth embodiment, subjects described herein (e.g., as in the first to ninth embodiments) are selected if the hippocampal volume is at least 5% lower than the baseline hippocampal volume, and the baseline hippocampal volume is obtained within 12 months prior to selection. Alternatively, as part of a tenth embodiment, subjects described herein (e.g., as in the first to ninth embodiments) are selected if the hippocampal volume is at least 10% lower than the baseline hippocampal volume, and the baseline hippocampal volume is obtained within 24 months prior to selection.

In an eleventh embodiment, subjects described herein (e.g., as in the first to ninth embodiments) are selected if the hippocampal volume is at least 12%, at least 11%, at least 10%, at least 9%, at least 8%, at least 7%, at least 6%, at least 5%, at least 4%, or at least 3% below the average age-adjusted hippocampal volume determined in a population of normal humans. Alternatively, as part of an eleventh embodiment, subjects described herein (e.g., as in the first to ninth embodiments) are selected if the hippocampal volume is 12%, 11%, 10%, 9%, 8%, 7%, 6%, 5%, 4%, or 3% below the average age-adjusted hippocampal volume determined in a population of normal humans. In another alternative, as part of an eleventh embodiment, subjects described herein (e.g., as in the first to ninth embodiments) are selected if the hippocampal volume is at least 10% below the average age-adjusted hippocampal volume determined in a population of normal humans. In another alternative, as part of an eleventh embodiment, subjects described herein (e.g., as in the first to ninth embodiments) are selected if the hippocampal volume is at least 7% below the average age-adjusted hippocampal volume determined in a population of normal humans. In yet another alternative, as part of an eleventh embodiment, subjects described herein (e.g., as in the first to ninth embodiments) are selected if the hippocampal volume is at least 5% below the average age-adjusted hippocampal volume determined in a population of normal humans. In yet another alternative, as part of an eleventh embodiment, subjects described herein (e.g., as in the first to ninth embodiments) are selected if the hippocampal volume is at least 4% below the average age-adjusted hippocampal volume determined in a population of normal humans

In a twelfth embodiment, subjects described herein (e.g., as in the eleventh embodiment) are selected if the hippocampal volume is less than 6.40 $cm^3$, less than 6.30 $cm^3$, less than 6.20 $cm^3$, less than 6.10 $cm^3$, less than 6.0 $cm^3$, less than 5.97 $cm^3$, less than 5.95 $cm^3$, less than 5.93 $cm^3$, less than 5.90 $cm^3$, less than 5.87 $cm^3$, less than 5.85 $cm^3$, less than 5.83 $cm^3$, less than 5.80 $cm^3$, less than 5.77 $cm^3$, less than 5.75 $cm^3$, less than 5.73 $cm^3$, less than 5.70 $cm^3$, less than 5.67 $cm^3$, less than 5.65 $cm^3$, less than 5.63 $cm^3$, less than 5.60 $cm^3$, less than 5.57 $cm^3$, less than 5.55 $cm^3$, less than 5.53 $cm^3$, less than 5.50 $cm^3$, less than 5.47 $cm^3$, less than 5.45 $cm^3$, less than 5.43 $cm^3$, less than 5.40 $cm^3$, less than 5.37 $cm^3$, less than 5.35 $cm^3$, less than 5.33 $cm^3$, less than 5.30 $cm^3$, less than 5.27 $cm^3$, less than 5.25 $cm^3$, less than 5.23 $cm^3$, less than 5.20 $cm^3$, less than 5.17 $cm^3$, less than 5.15 $cm^3$, less than 5.13 $cm^3$, less than 5.10 $cm^3$, less than 5.07 $cm^3$ less than 5.05 $cm^3$, less than 5.03 $cm^3$, less than 5.00 $cm^3$, less than 4.97 $cm^3$, less than 4.95 $cm^3$, less than 4.93 $cm^3$, less than 4.90 $cm^3$, less than 4.87 $cm^3$, less than 4.85 $cm^3$, less than 4.83 $cm^3$, less than 4.80 $cm^3$, less than 4.77 $cm^3$, less than 4.75 $cm^3$, less than 4.73 $cm^3$, less than 4.70 $cm^3$, less than 4.67$cm^3$, less than 4.65 $cm^3$, less than 4.63 $cm^3$, or less than 4.60 $cm^3$. Alternatively, as part of a twelfth embodiment, subjects described herein (e.g., as in the eleventh embodiment) are selected if the hippocampal volume ranges from 4.60 $cm^3$ to 6.40 $cm^3$, e.g., from 4.60 $cm^3$ to 6.20 $cm^3$, from 4.60 $cm^3$ to 6.00 $cm^3$, from 4.60 $cm^3$ to 5.80 $cm^3$, from 4.60 $cm^3$ to 5.50 $cm^3$, from 4.60 $cm^3$ to 5.30 $cm^3$, from 4.60 $cm^3$ to 5.10 $cm^3$, from 4.60 $cm^3$ to 4.90 $cm^3$, or from 4.70 $cm^3$ to 4.90 $cm^3$. Alternatively, as part of a twelfth embodiment, subjects described herein (e.g., as in the eleventh embodiment) are selected if the hippocampal volume is less 6.4 $cm^3$. Alternatively, as part of a twelfth embodiment, subjects described herein (e.g., as in the eleventh embodiment) are selected if the hippocampal volume is less 4.83 $cm^3$. Alternatively, as part of a twelfth embodiment, subjects described herein (e.g., as in the eleventh

embodiment) are selected if the hippocampal volume is less 4.60 $cm^3$.

In a thirteenth embodiment, subjects described herein (e.g., as in the ninth embodiment) are selected if the cortical thickness is at least 2% less than the baseline cortical thickness, and the baseline cortical thickness is obtained within 12 months prior to selection. Alternatively, as part of a thirteenth embodiment, subjects described herein (e.g., as in the ninth embodiment) are selected if the cortical thickness is at least 3% less than the baseline cortical thickness, and the baseline cortical thickness is obtained within 12 months prior to selection. Alternatively, as part of a thirteenth embodiment, subjects described herein (e.g., as in the ninth embodiment) are selected if the cortical thickness is at least 4% less than the baseline cortical thickness, and the baseline cortical thickness is obtained within 24 months prior to selection. Alternatively, as part of a thirteenth embodiment, subjects described herein (e.g., as in the ninth embodiment) are selected if the cortical thickness is at least 6% less than the baseline cortical thickness, and the baseline cortical thickness is obtained within 24 months prior to selection.

In a fourteenth embodiment, subjects described herein (e.g., as in the ninth embodiment) are selected if the cortical thickness is less than 2.61 mm, less than 2.60 mm, less than 2.59 mm, less than 2.58 mm, less than 2.57 mm, less than 2.56 mm, less than 2.55 mm, less than 2.54 mm, less than 2.53 mm, less than 2.52 mm, less than 2.51 mm, less than 2.50 mm, less than 2.49 mm, less than 2.48 mm, less than 2.47 mm, less than 2.46 mm, or less than 2.45 mm. Alternatively, as part of a fourteenth embodiment, subjects described herein (e.g., as in the ninth embodiment) are selected if the cortical thickness is between 2.45 mm and 2.61 mm e.g., between 2.45 mm and 2.59 mm, between 2.45 mm and 2.57 mm, or between 2.45 mm and 2.55 mm. Alternatively, as part of a fourteenth embodiment, subjects described herein (e.g., as in the ninth embodiment) are selected if the cortical thickness is less than 2.61 mm. Alternatively, as part of a fourteenth embodiment, subjects described herein (e.g., as in the ninth embodiment) are selected if the cortical thickness is less than 2.45 mm.

In a fifteenth embodiment, subjects described herein (e.g., as in the first to fourteenth embodiments) are determined to have an APOE3/APOE4 genotype, wherein the subjects are selected only if the subjects are further determined to be positive for amyloid deposits in the brain.

In a sixteenth embodiment, subjects described herein (e.g., as in the first to fourteenth embodiments) are determined to have an APOE3/APOE4 genotype and are selected 1) only if the subjects are further determined to be positive for amyloid deposits in the brain; 2) if the hippocampal volume is at least 5% lower than the baseline hippocampal volume; and 3) the baseline hippocampal volume is obtained within 12 months prior to the selection. Alternatively, as part of a sixteenth embodiment, subjects described herein (e.g., as in the first to fourteenth embodiments) are determined to have an APOE3/APOE4 genotype and are selected 1) only if the subjects are further determined to be positive for amyloid deposits in the brain; 2) if the hippocampal volume is at least 10% lower than the baseline hippocampal volume; and 3) the baseline hippocampal volume is obtained within 24 months prior to the selection.

In a seventeenth embodiment, subjects described herein (e.g., as in the first to fourteenth embodiments) are determined to have an APOE3/APOE4 genotype and are selected only if the subjects are further determined to be positive for amyloid deposits in the brain; and are selected if the hippocampal volume is at least 12%, at least 11%, at least 10%, at least 9%, at least 8%, at least 7%, at least 6%, at least 5%, or at least 4% below the average age-adjusted hippocampal volume determined in a population of normal humans. Alternatively, as part of a seventeenth embodiment, subjects described herein (e.g., as in the first to fourteenth embodiments) are determined to have an APOE3/APOE4 genotype and are selected only if the subjects are further determined to be positive for amyloid deposits in the brain; and are selected if the hippocampal volume is 12%, 11%, 10%, 9%, 8%, 7%, 6%, 5%, or 4% below the average age-adjusted hippocampal volume determined in a population of normal humans. In another alternative, as part of a seventeenth embodiment, subjects described herein (e.g., as in the first to fourteenth embodiments) are determined to have an APOE3/APOE4 genotype and are selected only if the subjects are further determined to be positive for amyloid deposits in the brain; and are selected if the hippocampal volume is at least 10% below the average age-adjusted hippocampal volume determined in a population of normal humans. In another alternative, as part of a seventeenth embodiment, subjects described herein (e.g., as in the first to fourteenth embodiments) are determined to have an APOE3/APOE4 genotype and are selected only if the subjects are further determined to be positive for amyloid deposits in the brain; and are selected if the hippocampal volume is at least 5% below the average age-adjusted hippocampal volume determined in a population of normal humans.

In an eighteenth embodiment, subjects described herein (e.g., as in the seventeenth embodiment) are selected if the hippocampal volume is less than 5.40 $cm^3$, less than 5.37 $cm^3$, less than 5.33 $cm^3$, less than 5.30 $cm^3$, less than 5.27 $cm^3$, less than 5.23 $cm^3$, less than 5.20 $cm^3$, less than 5.17 $cm^3$, less than 5.15 $cm^3$, less than 5.12 $cm^3$, less than 5.10 $cm^3$, less than 5.07 $cm^3$, less than 5.05 $cm^3$, less than 5.02 $cm^3$, less than 5.00 $cm^3$, less than 4.97 $cm^3$, less than 4.95 $cm^3$, less than 4.92 $cm^3$, less than 4.90 $cm^3$, less than 4.87 $cm^3$, less than 4.85 $cm^3$, less than 4.82 $cm^3$, less than 4.80 $cm^3$, less than 4.77 $cm^3$, less than 4.75 $cm^3$, less than 4.72 $cm^3$, or less than 4.70 $cm^3$. Alternatively, as part of an eighteenth embodiment, subjects described herein (e.g., as in the seventeenth embodiment) are selected if the hippocampal volume ranges from 4.70 $cm^3$ to 5.40 $cm^3$, from 4.70 $cm^3$ to 5.20 $cm^3$, or from 5.05 $cm^3$ to 5.45 $cm^3$, from 5.10 $cm^3$ to 5.40 $cm^3$. Alternatively, as part of an eighteenth embodiment, subjects described herein (e.g., as in the fourteenth embodiment) are selected if the hippocampal volume is less 4.70 $cm^3$. Alternatively, as part of an eighteenth embodiment, subjects described herein (e.g.,

as in the fourteenth embodiment) are selected if the hippocampal volume is less 5.20 $cm^3$. Alternatively, as part of an eighteenth embodiment, subjects described herein (e.g., as in the fourteenth embodiment) are selected if the hippocampal volume is less 5.40 $cm^3$. Alternatively, as part of an eighteenth embodiment, subjects described herein (e.g., as in the fourteenth embodiment) are selected if the hippocampal volume is less 5.10 $cm^3$.

In a nineteenth embodiment, subjects described herein (e.g., as in the first to fourteenth embodiments) are determined to have an APOE3/APOE4 genotype, wherein the subjects are selected only if the subjects are further determined to be positive for amyloid deposits in the brain, and wherein the subject is selected if the cortical thickness is at least 3% less than the baseline cortical thickness, and the baseline cortical thickness is obtained within 12 months prior to the selection. Alternatively, as part of a nineteenth embodiment, subjects described herein (e.g., as in the first to fourteenth embodiments) are determined to have an APOE3/APOE4 genotype, wherein the subjects are selected only if the subjects are further determined to be positive for amyloid deposits in the brain, and wherein the subject is selected if the cortical thickness is at least 6% less than the baseline cortical thickness, and the baseline cortical thickness is obtained within 24 months prior to the selection.

In a twentieth embodiment, subjects described herein (e.g., as in the first to fourteenth embodiments) are determined to have an APOE3/APOE4 genotype, wherein the subjects are selected only if the subjects are further determined to be positive for amyloid deposits in the brain, and wherein the subjects are selected if the cortical thickness is less than 2.60 mm, less than 2.59 mm, less than 2.58 mm, less than 2.57 mm, less than 2.56 mm, less than 2.55 mm, less than 2.54 mm, less than 2.53 mm, less than 2.52 mm, less than 2.51 mm, less than 2.50 mm, less than 2.49 mm, less than 2.48 mm, less than 2.47 mm, less than 2.46 mm, less than 2.45 mm, less than 2.44 mm, less than 2.43 mm, or less than 2.42 mm. Alternatively, as part of a twentieth embodiment, subjects described herein (e.g., as in the first to fourteenth embodiments) are determined to have an APOE3/APOE4 genotype, wherein the subjects are selected only if the subjects are further determined to be positive for amyloid deposits in the brain, and wherein the subjects are selected if the cortical thickness is between 2.42 mm and 2.60 mm e.g., between 2.45 mm and 2.60 mm, between 2.47 mm and 2.59 mm, or between 2.48 mm and 2.58 mm. Alternatively, as part of a twentieth embodiment, subjects described herein (e.g., as in the first to fourteenth embodiments) are determined to have an APOE3/APOE4 genotype, wherein the subjects are selected only if the subjects are further determined to be positive for amyloid deposits in the brain, and wherein the subjects are selected if the cortical thickness is less than 2.60 mm. Alternatively, as part of a twentieth embodiment, subjects described herein (e.g., as in the first to fourteenth embodiments) are determined to have an APOE3/APOE4 genotype, wherein the subjects are selected only if the subjects are further determined to be positive for amyloid deposits in the brain, and wherein the subjects are selected if the cortical thickness is less than 2.42 mm.

In a twenty-first embodiment, subjects described herein (e.g., as in the first to fourteenth embodiments) are determined to have an APOE2/APOE4 genotype, wherein the subjects are selected only if the subjects are further determined to be positive for amyloid deposits in the brain.

In a twenty-second embodiment, subjects described herein (e.g., as in the first to fourteenth embodiments) are determined to have an APOE2/APOE4 genotype and are selected only if the subjects are further determined to be positive for amyloid deposits in the brain; and if the hippocampal volume is at least 4% lower than the baseline hippocampal volume, and the baseline hippocampal volume is obtained within 12 months prior to the selection. Alternatively, as part of a twenty-second embodiment, subjects described herein (e.g., as in the first to fourteenth embodiments) are determined to have an APOE2/APOE4 genotype and are selected only if the subjects are further determined to be positive for amyloid deposits in the brain; and if the hippocampal volume is at least 7% lower than the baseline hippocampal volume, and the baseline hippocampal volume is obtained within 36 months prior to the selection.

In a twenty-third embodiment, subjects described herein (e.g., as in the first to fourteenth embodiments) are determined to have an APOE2/APOE4 genotype and are selected only if the subjects are further determined to be positive for amyloid deposits in the brain; and are selected if the hippocampal volume is at least 10%, at least 9%, at least 8%, at least 7%, at least 6%, at least 5%, at least 4%, or at least 3% below the average age-adjusted hippocampal volume determined in a population of normal humans. Alternatively, as part of a twenty-third embodiment, subjects described herein (e.g., as in the first to fourteenth embodiments) are determined to have an APOE2/APOE4 genotype and are selected only if the subjects are further determined to be positive for amyloid deposits in the brain; and are selected if the hippocampal volume is 10%, 9%, 8%, 7%, 6%, 5%, 4%, or 3% below the average age-adjusted hippocampal volume determined in a population of normal humans. In another alternative, as part of a twenty-third embodiment, subjects described herein (e.g., as in the first to fourteenth embodiments) are determined to have an APOE2/APOE4 genotype and are selected only if the subjects are further determined to be positive for amyloid deposits in the brain; and are selected if the hippocampal volume is at least 7% below the average age-adjusted hippocampal volume determined in a population of normal humans. In another alternative, as part of a twenty-third embodiment, subjects described herein (e.g., as in the first to fourteenth embodiments) are determined to have an APOE2/APOE4 genotype and are selected only if the subjects are further determined to be positive for amyloid deposits in the brain; and are selected if the hippocampal volume is at least 4% below the average age-adjusted hippocampal volume determined in a population of normal humans.

In a twenty-fourth embodiment, subjects described herein (e.g., as in the twenty-third embodiment) are selected

if the hippocampal volume is less than less than 6.40 $cm^3$, less than 6.37 $cm^3$, less than 6.35 $cm^3$, less than 6.32 $cm^3$, less than 6.31 $cm^3$, less than 6.30 $cm^3$, less than 6.27 $cm^3$, less than 6.25 $cm^3$, less than 6.22 $cm^3$, less than 6.20 $cm^3$, less than 6.17 $cm^3$, less than 6.15 $cm^3$, less than 6.12 $cm^3$, less than 6.10 $cm^3$, less than 6.08 $cm^3$, less than 6.07 $cm^3$, less than 6.06 $cm^3$, less than 6.05 $cm^3$, less than 6.04 $cm^3$, less than 6.03 $cm^3$, less than 6.02 $cm^3$, less than 6.10 $cm^3$, less than 6.00 $cm^3$, less than 5.97 $cm^3$, or less than 5.95 $cm^3$. Alternatively, as part of a twenty-fourth embodiment, subjects described herein (e.g., as in the twenty-third embodiment) are selected if the hippocampal volume ranges from 5.95 $cm^3$ to 6.40 $cm^3$ e.g., from 6.00 $cm^3$ to 6.37 $cm^3$, from 6.02 $cm^3$ to 6.35 $cm^3$, from 6.05 $cm^3$ to 6.33 $cm^3$, or from 6.07 $cm^3$ to 6.31 $cm^3$. Alternatively, as part of a twenty-fourth embodiment, subjects described herein (e.g., as in the twenty-third embodiment) are selected if the hippocampal volume is less 6.10 $cm^3$. Alternatively, as part of a twenty-fourth embodiment, subjects described herein (e.g., as in the twenty-third embodiment) are selected if the hippocampal volume is less 5.95 $cm^3$. Alternatively, as part of a twenty-fourth embodiment, subjects described herein (e.g., as in the twenty-third embodiment) are selected if the hippocampal volume is less 6.31 $cm^3$. Alternatively, as part of a twenty-fourth embodiment, subjects described herein (e.g., as in the twenty-third embodiment) are selected if the hippocampal volume is less 6.07 $cm^3$.

In a twenty-fifth embodiment, subjects described herein (e.g., as in the first to fourteenth embodiments) are determined to have an APOE2/APOE4 genotype, wherein the subjects are selected only if the subjects are further determined to be positive for amyloid deposits in the brain, and wherein the subject is selected if the cortical thickness is at least 2% less than the baseline cortical thickness, and the baseline cortical thickness is obtained within 12 months prior to the selection. Alternatively, as part of a twenty-fifth embodiment, subjects described herein (e.g., as in the first to fourteenth embodiments) are determined to have an APOE2/APOE4 genotype, wherein the subjects are selected only if the subjects are further determined to be positive for amyloid deposits in the brain, and wherein the subject is selected if the cortical thickness is at least 4% less than the baseline cortical thickness, and the baseline cortical thickness is obtained within 36 months prior to the selection.

In a twenty-sixth embodiment, subjects described herein (e.g., as in the first to fourteenth embodiments) are determined to have an APOE2/APOE4 genotype, wherein the subjects are selected only if the subjects are further determined to be positive for amyloid deposits in the brain, and wherein the subjects are selected if the cortical thickness is less than 2.63 mm, less than 2.62 mm, less than 2.61 mm, less than 2.60 mm, less than 2.59 mm, less than 2.58 mm, less than 2.57 mm, less than 2.56 mm, less than 2.55 mm, less than 2.54 mm, less than 2.53 mm, less than 2.52 mm, less than 2.51 mm, less than 2.50 mm, less than 2.48 mm, less than 2.46 mm, or less than 2.43 mm. Alternatively, as part of a twenty-sixth embodiment, subjects described herein (e.g., as in the first to fourteenth embodiments) are determined to have an APOE2/APOE4 genotype, wherein the subjects are selected only if the subjects are further determined to be positive for amyloid deposits in the brain, and wherein the subjects are selected if the cortical thickness is between 2.45 and 2.63 mm e.g., between 2.50 and 2.63 mm, between 2.46 and 2.60 mm, between 2.46 mm and 2.58 mm, between 2.46 and 2.57 mm, or between 2.47 and 2.57 mm. Alternatively, as part of a twenty-sixth embodiment, subjects described herein (e.g., as in the first to fourteenth embodiments) are determined to have an APOE2/APOE4 genotype, wherein the subjects are selected only if the subjects are further determined to be positive for amyloid deposits in the brain, and wherein the subjects are selected if the cortical thickness is less than 2.63 mm. Alternatively, as part of a twenty-sixth embodiment, subjects described herein (e.g., as in the first to fourteenth embodiments) are determined to have an APOE2/APOE4 genotype, wherein the subjects are selected only if the subjects are further determined to be positive for amyloid deposits in the brain, and wherein the subjects are selected if the cortical thickness is less than 2.50 mm.

In a twenty-seventh embodiment, subjects described herein (e.g., as in the first to fourteenth embodiments) are determined to have an APOE4/APOE4 genotype and are selected only if the subject's hippocampal volume is at least 5% lower than the baseline hippocampal volume, and the baseline hippocampal volume is obtained within 12 months prior to the selection. Alternatively, as part of a twenty-seventh embodiment, subjects described herein (e.g., as in the first to fourteenth embodiments) are determined to have an APOE4/APOE4 genotype and are selected only if the subjects are further determined to be positive for amyloid deposits in the brain; and if the hippocampal volume is at least 10% lower than the baseline hippocampal volume, and the baseline hippocampal volume is obtained within 36 months prior to the selection.

In a twenty-eighth embodiment, subjects described herein (e.g., as in the first to fourteenth embodiments) are determined to have an APOE4/APOE4 genotype and are selected only if the subject's hippocampal volume is at least 12%, at least 11%, at least 10%, at least 9%, at least 8%, at least 7%, at least 6%, at least 5%, or at least 4% below the average age-adjusted hippocampal volume determined in a population of normal humans. Alternatively, as part of a twenty-eighth embodiment, subjects described herein (e.g., as in the first to fourteenth embodiments) are determined to have an APOE2/APOE4 genotype and are selected only if the subject's hippocampal volume is 12%, 11%, 10%, 9%, 8%, 7%, 6%, 5%, or 4% below the average age-adjusted hippocampal volume determined in a population of normal humans. In another alternative, as part of a twenty-eighth embodiment, subjects described herein (e.g., as in the first to fourteenth embodiments) are determined to have an APOE4/APOE4 genotype and are selected only if the subjects hippocampal volume is at least 10% below the average age-adjusted hippocampal volume determined in a population of normal humans. In another alternative, as part of a twenty-eighth embodiment, subjects described herein (e.g., as in the first to

fourteenth embodiments) are determined to have an APOE4/APOE4 genotype and are selected only if the subjects are further determined to be positive for amyloid deposits in the brain; and are selected if the hippocampal volume is at least 5% below the average age-adjusted hippocampal volume determined in a population of normal humans.

In a twenty-ninth embodiment, subjects described herein (e.g., as in the first to fourteenth embodiments) are selected only if the hippocampal volume is less than 5.20 $cm^3$, less than 5.10 $cm^3$, less than 5.00 $cm^3$, less than 4.95 $cm^3$, less than 4.90 $cm^3$, less than 4.85 $cm^3$, less than 4.80 $cm^3$, less than 4.75 $cm^3$, less than 4.70 $cm^3$, less than 4.65 $cm^3$, or less than 4.60 $cm^3$. Alternatively, as part of a twenty-ninth embodiment, subjects described herein (e.g., as in the first to fourteenth embodiments) are selected only if the hippocampal volume ranges from 4.60 $cm^3$ to 5.20 $cm^3$. Alternatively, as part of a twenty-ninth embodiment, subjects described herein (e.g., as in the first to fourteenth embodiments) are selected only if the hippocampal volume is less 5.20 $cm^3$.

In a thirtieth embodiment, subjects described herein (e.g., as in the first to fourteenth embodiments) are determined to have an APOE4/APOE4 genotype, wherein the subject is selected only if the subject's cortical thickness is at least 3% less than the baseline cortical thickness, and the baseline cortical thickness is obtained within 12 months prior to the selection. Alternatively, as part of a thirtieth embodiment, subjects described herein (e.g., as in the first to fourteenth embodiments) are determined to have an APOE4/APOE4 genotype, wherein the subject is selected if the cortical thickness is at least 6% less than the baseline cortical thickness, and the baseline cortical thickness is obtained within 36 months prior to the selection.

In a thirty-first embodiment, subjects described herein (e.g., as in the first to fourteenth embodiments) are determined to have an APOE4/APOE4 genotype, wherein the subject is selected only if the subject's cortical thickness is less than 2.57 mm, less than 2.56 mm, less than 2.55 mm, less than 2.54 mm, less than 2.53 mm, less than 2.52 mm, less than 2.51 mm, less than 2.50 mm, less than 2.49 mm, less than 2.48 mm, less than 2.47 mm, less than 2.46 mm, less than 2.45 mm, less than 2.44 mm, less than 2.43 mm, less than 2.42 mm, less than 2.41 mm, or less than 2.40 mm. Alternatively, as part of a thirty-first embodiment, subjects described herein (e.g., as in the first to fourteenth embodiments) are determined to have an APOE4/APOE4 genotype, wherein the subject is selected only if the subject's cortical thickness is less than 2.57 mm.

In a thirty-second embodiment, subjects described herein (e.g., as in the first to thirty-first embodiments) are 50 years of age or greater at the time of the selection.

In a thirty-third embodiment, subjects described herein (e.g., as in the first to thirty-second embodiments) are administered a tramiprosate prodrug of the formula:

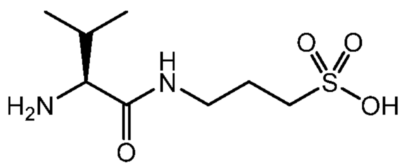

,

or a pharmaceutically acceptable salt thereof.

In a thirty-fourth embodiment, subjects described herein (e.g., as in the first to thirty-second embodiments) are administered a tramiprosate metabolite of the formula:

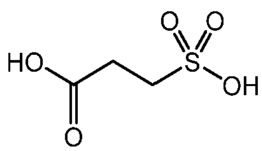

,

or a pharmaceutically acceptable salt thereof.

In a thirty-fifth embodiment, subjects described herein (e.g., as in the first to thirty-fourth embodiments) are determined to be asymptomatic for Alzheimer's disease if the subject scores 28 or higher on a MMSE.

In a thirty-sixth embodiment, one or both of hippocampal volume and cortical thickness described herein (e.g., as in the first to thirty-fifth embodiments) is measured by MRI.

In a thirty-seventh embodiment, amyloid deposits as described herein (e.g., in the seventh to thirty-sixth embodiments) are detected by PET imaging or cerebral spinal fluid biomarkers.

In a thirty-eighth embodiment, subjects described herein (e.g., as in the first to fourteenth embodiments) are determined to have an APOE4/APOE4 genotype and have at least one of 1) an MMSE score of 28 or greater, 2) a 5% or greater decline over 12 months in hippocampus volume; 3) a 10% or greater 10% decline over 24 months in hippocampus volume; 4) a 3% or greater decline in cortical thickness over 12 months; or 5) a 6% or greater decline in cortical thickness

over 24 months. Alternatively or in addition to, the hippocampus volume in the subject may be less than 5.37 $cm^3$ and/or the cortical thickness is less than 2.62 mm.

In a thirty-ninth embodiment, subjects described herein (e.g., as in the first to fourteenth embodiments) are determined to have an APOE3/APOE4 genotype and have at least one of 1) an MMSE score of greater than 28; 2) evidence of brain amyloid by PET imaging or CSF biomarker; 3) a 5% or greater decline over 12 months in hippocampus volume; 4) a 10% or greater 10% decline over 24 months in hippocampus volume; 5) a 3% or greater decline in cortical thickness over 12 months; or 6) a 6% or greater decline in cortical thickness over 24 months. Alternatively or in addition to, the hippocampus volume may be less than 5.66 $cm^3$ and/or the cortical thickness is less than 2.64 mm.

In a fortieth embodiment, subjects described herein (e.g., as in the first to fourteenth embodiments) are determined to have an APOE2/APOE4 genotype and have at least one of 1) an MMSE score of greater than 28; 2) evidence of brain amyloid by PET imaging or CSF biomarker; 3) a 4% or greater decline in hippocampus volume over 12 months; 4) a 7% or greater decline over 24-36 months in hippocampus volume; 5) a 2% or more decline over 12 months in cortical thickness; or 6) a 4% or more decline over 24-36 months in cortical thickness. Alternatively or in addition to, the hippocampus volume may be less than 6.57 $cm^3$ and/or the cortical thickness is less than 2.66 mm.

Other AD drugs are contemplated for use in the present methods. Such drugs include, but are not limited to, anti-amyloid drugs, anti-tau drugs, anti-inflammatory drugs, and other AD targeted drugs, including combination therapies. Anti-amyloid drugs are those which inhibit to formation of amyloid directly or indirectly and can include e.g., those drugs which inhibit amyloid precursors or the enzymes involved in their proteolytic processing. Examples of anti-amyloid drugs include, but are not limited to, scyllo-inositol, BAN2401, CAD106 3, Gantenerumab, Elenbecestat (E2609), CNP520 (AMG520), CNP520, ACI-24 2, ABvac40, LY3002813, UB-311, PQ912, LY3372993, KHK6640 1, Lu AF20513, and MEDI1814. Anti-tau drugs are those which modulate tau proteins and, in some aspects, prevent the formation of neurofibrillary tangles in the brain. Examples of anti-tau drugs include, but are not limited to, LMTX, ABBV-8E12, AADvac-1, BIIB092, LY3303560, RG6100, Nilotinib, ACI-35, BIIB080/IONIS-MAPTrx, BIIB076, JNJ-63733657, PU-AD, and TPI287 1. Anti-inflammatory drugs are those which have effects on inflammation in the brain and, in some aspects, reduce the production of residues of the β amyloid peptide. Examples of anti-inflammatory drugs include, but are not limited to, Masitinib, ALZT-OP1, GV-971, COR388, GM-CSF, Telmisartan, PTI-125, Neflamapimod (VX-745), Azeliragon (TTP488), DNL747, GC021109, AL002, and XPro1595. Other AD targeted drugs include those described in "Hope remains on Alzheimer's disease treatments: our in-depth look at biopharma's clinical pipeline" Biotechnology industry update June 28, 2019, Sumant Kulkarni et al., Canaccord Genuity Capital Markets.

In a forty-first embodiment, hippocampal volume and cortical thickness measurements are used together to determine if a subject is to be treated by any of the agents set forth herein. In some aspects, the subject may be a carrier or non-carrier of at least one APOE4 allele. For example, in one aspect of this forty-first embodiment the subject may be a carrier of at least one APOE4. **In** another aspect of this forty-first embodiment, the subject comprises an APOE4/4 genotype. **In** another aspect of this forty-first embodiment, the subject comprises an APOE3/4 genotype. **In** one aspect of this forty-first embodiment, hippocampal volume and cortical thickness measurements of a subject described herein are summed after one or both of these parameters is optionally adjusted by a weighting factor, and that weighted sum is then compared to a threshold value. Subjects whose weighted sum are below a threshold value for such weighted sums are then selected for treatment with tramiprosate, a pharmaceutically acceptable salt of tramiprosate, a tramiprosate prodrug, a tramiprosate derivative, or an active tramiprosate metabolite. In certain aspects of this forty-first embodiment, the weighting factor will be determined by measuring both hippocampal volume and cortical thickness in a normal (non-AD, non-MCI) population and determining how to best weight those values so that a plot of the weighted sum versus age shows a linear or quasi-linear relationship.

In these aspects, the threshold will vary by the age of the subject and the threshold value for a test subject will therefore also be based on the subject's age. In certain aspects of this forty-first embodiment, the weighting factor will simply place both parameters at equivalent numerical values before summing. **In** certain aspects of this forty-first embodiment, the weighting factor will take into account whether a subject is a carrier for least one APOE4 allele as defined herein. For example, if the average hippocampal volume in normal subjects of a specific age is 7 $cm^3$ and the average cortical thickness in such subjects is 3 mm, the cortical thickness value will be weighted by a factor of 7/3 in order to place the numerical values of cortical thickness on an equivalent level as the numerical values of hippocampal volumes.

In still other alternate aspects of the forty-first embodiment a subject is selected for treatment if: a) their hippocampal volume is below a threshold; and b) the ratio of their hippocampal volume/cortical thickness above a threshold (or the ratio of their cortical thickness/hippocampal volume is below is threshold). In still other alternate aspects of the forty-first embodiment a subject is selected for treatment if: a) their hippocampal volume is below a threshold; b) the ratio of their hippocampal volume/cortical thickness above a threshold (or the ratio of their cortical thickness/hippocampal volume is below is threshold); and they are a carrier of at least one APOE allele (e.g., comprise an APOE4/4 or APOE3/4 genotype). In still other alternate aspects of the forty-first embodiment a subject is selected for treatment if: a) their cortical thickness is below a threshold; and b) the ratio of their cortical thickness/hippocampal volume is above a threshold (or their hippocampal volume/cortical thickness is above a threshold). In still other alternate aspects of the forty-first embodiment a

subject is selected for treatment if: a) their cortical thickness is below a threshold; b) the ratio of their cortical thickness/hippocampal volume is above a threshold (or their hippocampal volume/cortical thickness is above a threshold); and they are a carrier of at least one APOE allele (e.g., they comprise an APOE4/4 or APOE3/4 genotype). It will be apparent to those of ordinary skill in the art, that the goal of these aspects is to ensure that a subject is not selected on only one of hippocampal volume or cortical thickness without also demonstrating at least some reduction in the other parameter as compared to normal controls. However, it should also be clear that in these aspects, the subject need not have both hippocampal volume and cortical thickness below their respective thresholds in order to select that subject for treatment.

In a forty-second embodiment, hippocampal volume and/or cortical thickness measurements are used together with one or more serum AD-related tests to select subjects for treatment with tramiprosate, a pharmaceutically acceptable salt of tramiprosate, a tramiprosate prodrug, a tramiprosate derivative, or an active tramiprosate metabolite. In some aspects of a forty-second embodiment, hippocampal volume and/or cortical thickness measurements are used together with one or more with serum AD-related tests to select subjects for treatment with tramiprosate, a pharmaceutically acceptable salt of tramiprosate, a tramiprosate prodrug, a tramiprosate derivative, or an active tramiprosate metabolite wherein the subject has at least one APOE4 allele (e.g., they comprise an APOE4/4 or APOE3/4 genotype). In some aspects of this forty-second embodiment, the serum AD-related test is one or more of phosphorylated tau 181 (TK Karikari et al., Lancet Neurol 2020; 19: 422-33; S Janelidze et al., Nature Med 2020; 26:379-86; INNOTEST® PHOSPHO-TAU(181P), Fujirebio Europe), phosphorylated tau 217 (Nicolas R. Barthélemy et al., Alzheimer's Research & Therapy volume 12, Article number: 26 (2020)) neurofilament light chain (NfL; NJ Ashton et al., Acta Neuropath Comm (2019) 7:5; N Mattsson et al., JAMA Neurol 2017, 74(5):557-66), total tau protein (INNOTEST® hTAU-Ag); Aβ1-42 (INNOTEST® β-AMYLOID(1-42)), or Aβ1-42/Aβ1-40 ratio (INNOTEST® β-AMYLOID(1-42), β-AMYLOID(1-40)). In some aspects of this forty-second embodiment, a subject would require both (a) a hippocampal volume and/or cortical thickness below a threshold level; and (b) serum AD biomarker above (for assays where a higher level indicates AD or MCI such as phospho-tau 181, total tau protein, or NfL), or below (for assays where a lower level indicates AD or MCI such as Aβ1-42 or Aβ1-42/Aβ1-40 ratio) a threshold level in order to be selected for treatment. In some aspects of this forty-second embodiment, a subject would require both (a) a hippocampal volume and/or cortical thickness below a threshold level; and (b) serum AD biomarker above (for assays where a higher level indicates AD or MCI such as phospho-tau 181, total tau protein, or NfL), or below (for assays where a lower level indicates AD or MCI such as Aβ1-42 or Aβ1-42/Aβ1-40 ratio) a threshold level; and have at least one APOE4 allele (e.g., they comprise an APOE4/4 or APOE3/4 genotype) in order to be selected for treatment. In some aspects of this forty-second embodiment, the serum AD-related test is one or more of phosphorylated tau 181, phosphorylated tau 217, or NfL.

In other aspects of the forty-second embodiment, a single value reflecting both (a) hippocampal volume and/or cortical thickness; and (b) one or more serum AD biomarker levels would be obtained for a subject and the subject would be selected for treatment if that value was above (or below depending upon how the single value is obtained) a threshold level based on that same single value in a normal population. A single value for two or more parameters may be obtained by summing (after one or more of these parameters is optionally adjusted by a weighting factor), wherein all parameters whose lower values indicate MCI or AD (e.g., cortical thickness, hippocampal volume, serum Aβ1-42 levels, serum Aβ1-42/Aβ1-40 ratios) are assigned a positive value and all parameters whose higher values indicate MCI or AD (e.g., phospho-tau 181, total tau protein, NfL) are either expressed as their inverse or assigned a negative value.

This can be expressed by the following formulae wherein hippocampal volume (HV), cortical thickness (CT) and serum phospho-tau 181 (PT181) values are used to obtain a single value :

$$Vall = (CT * W_{\mathrm{CT}}) + (HV * W_{\mathrm{HV}}) - (PT181 * W_{\mathrm{PT181}})$$

or $Vall = (CT * W_{\mathrm{CT}}) + (HV * W_{\mathrm{HV}}) + \left(\frac{1}{PT181} * W_{\mathrm{PT181}}\right)$, wherein $V_{all}$ is the single value; $W_{CT}$ is the optional CT weighting factor; $W_{HV}$ is the optional HV weighting factor; and $W_{PT181}$ is the optional phospho-tau 181 weighting factor. Subjects whose weighted sum are below a threshold value for such weighted sum are then selected for treatment with tramiprosate, a pharmaceutically acceptable salt of tramiprosate, a tramiprosate prodrug, a tramiprosate derivative, or an active tramiprosate metabolite. As set forth in the forty-first embodiment, determining the various weighting factors is achieved after sampling a control population. The same result can also be achieved by utilizing the inverse value of all parameters whose lower values indicate MCI or AD or assigning those parameters a negative value; assigning all parameters whose higher values indicate MCI or AD a positive value; and selecting subjects whose weighted sum is above a threshold.

In still other aspects of the forty-second embodiment, a single value reflecting both (a) hippocampal volume and/or cortical thickness; and (b) one or more serum AD biomarker may be obtained by calculating a ratio (after one or more of these parameters is optionally adjusted by a weighting factor), wherein all parameters whose lower values indicate MCI or AD (e.g., cortical thickness, hippocampal volume, serum Aβ1-42 levels, serum Aβ1-42/Aβ1-40 ratios) are summed

in the denominator and all parameters whose higher values indicate MCI or AD (e.g., phospho-tau 181, total tau protein, NfL) are summed in the numerator. This can be expressed by the following formulae wherein hippocampal volume (HV), cortical thickness (CT) and serum phospho-tau 181 (PT181) values are used to obtain a single value :

$\text{Vall} = \frac{PT181 * W_{PT181}}{(CT * W_{CT}) + (HV * W_{HV})}$, wherein $V_{all}$ is the single value; $W_{CT}$ is the optional CT weighting factor; $W_{HV}$ is the optional HV weighting factor; and $W_{PT181}$ is the optional phospho-tau 181 weighting factor. Subjects whose weighted ratio are above a threshold value for such weighted ratio are then selected for treatment with tramiprosate, a pharmaceutically acceptable salt of tramiprosate, a tramiprosate prodrug, a tramiprosate derivative, or an active tramiprosate metabolite.

As set forth in the forty-first embodiment, determining the various weighting factors is achieved after sampling a control population. The same result can also be achieved by utilizing the inverse value of all parameters whose lower values indicate MCI or AD or assigning those parameters a negative value; and assigning all parameters whose higher values indicate MCI or AD a positive value and selecting subjects whose weighted sum is above a threshold.

## EXEMPLIFICATION

### APOE4/4 Homozygotes Show High Rates of Hippocampus Atrophy and Accelerated Loss of Cortical Thickness that Correlate with Cognitive Decline at Early Stages of Alzheimer's Disease

### A. Methods

AD NeuroImaging Initiative *(ADNI-1) Study Population*

The volumetric MRI (vMRI) and clinical datasets were from the ADNI-1 observational study which was designed to evaluate multiple biomarkers at early stages of the AD spectrum. See Jack CR Jr, Bennett DA, Blennow K, Carrillo MC, Dunn B, Haeberlein SB, et al. NIA-AA Research Framework: Toward a biological definition of Alzheimer's disease. Alzheimers Dement; 2018;14:535-562 and Petersen RC, Aisen PS, Beckett LA, Donohue MC, Gamst AC, Harvey DJ, et al. Alzheimer's Disease Neuroimaging Initiative (ADNI): clinical characterization. Neurology 2010;74:201-9. The biomarkers included vMRI biomarkers and CSF assays of amyloid and tau levels. ADNI-1 enrolled 255 cognitively normal elderly, 301 with late mild cognitive impairment (LMCI), and 166 with mild AD. Disease stage was based on the Mini-Mental State Examination (MMSE). Mild AD subjects had MMSE scores of 20-26, and LMCI had MMSE scores ≥ 24. The cognitively normal group had only five APOE4/4 subjects with serial MRI, and therefore was not included in this analysis.

*ADNI-1 MRI Dataset*

In the LMCI and mild AD groups, a total of 172 subjects had the genotypes of interest and serial MRIs. Serial MRIs were performed at baseline, 12 and 24 months [http://adni.loni.ucla.edu]. The distribution of subjects with serial MRIs according to genotype is shown in **Table 1.** A subset of subjects also had CSF assays for Aβ42 and tau at baseline. Of the APOE3/3 subjects with LMCI and mild AD, 56% and 74% respectively had CSF profiles consistent with AD pathology (CSF positive), while all APOE4/4 subjects (LMCI and mild AD) were CSF positive **(Table 1).** In the MRI dataset, the respective numbers of CSF positive LMCI and mild AD subjects were 28 and 12 for APOE3/3, and 17 and 15 APOE4/4 subjects.

**Table 1**

| Number of subjects in the LMCI and mild AD analysis groups (ADNI-1 study) | | | | |
|---|---|---|---|---|
| | | | **LMCI** N = 301 | **Mild AD** N = 166 |
| Genotype | APOE3/3 | APOE4/4 | APOE3/3 | APOE4/4 |
| Overall sample | 228 | 73 | 101 | 65 |
| MRI subgroup | 93 | 29 | 29 | 21 |
| CSF+ subgroup | 46 | 21 | 22 | 23 |

Abbreviations: LMCI, late mild cognitive impairment; ADNI-1, Alzheimer's Disease Neuroimaging Initiative 1; APOE, apolipoprotein E; MRI subgroup: subjects with serial magnetic resonance imaging. CSF+ subgroup: subjects had positive baseline cerebrospinal fluid amyloid assay.

*Volumetric MRI Analyses*

The vMRI data included 3D T1-weighted images, which were acquired at 1.5T, and consisted of MP RAGE (Siemens), 3D TFE (Philips) and 3D Fast SPGR (General Electric) pulse sequences, with a 1.25×1.25×1.2 $mm^3$ voxel resolution in a sagittal orientation. These analyses included assessments of total HV, whole brain volume, and cortical thickness. All vMRI data were processed centrally by BioClinica with fully-automated methods using FreeSurfer v5.3 for brain segmentation at baseline. See Fischl B, Salat DH, Busa E, Albert M, Dieterich M, Haselgrove C, et al. Whole brain segmentation: automated labeling of neuroanatomical structures in the human brain. Neuron 2002;33:341-55 and Fischl B, van der Kouwe A, Destrieux C, Halgren E, Ségonne F, Salat DH, et al. Automatically parcellating the human cerebral cortex. Cereb Cortex 2004;14:11-22. Volumes of whole brain (WBV), lateral ventricles (LVV) and total HV (HV = L + R) were derived. Volume changes at follow-up timepoints were assessed using Boundary Shift Integral. See Leung KK, Ridgway GR, Ourselin S, Fox NC. Consistent multi-time-point brain atrophy estimation from the boundary shift interval. NeuroImage 2012;59:3995-4005.

For ADNI data, cortical thickness was measured at baseline using FreeSurfer (Fischl 2002, 2004 cite above), a composite average thickness was derived as Mayo AD signature ROI. See Mayo index and Jack CR Jr, Wiste HJ, Weigand SD, Therneau TM, Lowe VJ, Knopman DS, et al. Defining imaging biomarker cut-points for brain aging and Alzheimer's disease. Alzheimers Dement 2017;13:205-216. Cortical thickness/Mayo Index changes were analyzed by a Jacobian-based method. See Schwarz CG, Gunter JL, Wiste HJ, Przybelski SA, Weigand SD, Ward CP, et al. A large-scale comparison of cortical thickness and volume methods for measuring Alzheimer's Disease Severity. Neuroimage 2016;11:802-81. Volumetric measures were adjusted for age, years of education, and head size.

APOE4/4 effects on imaging endpoints were assessed at Baseline and Month 24 using a two-sample t-test, after adjusting for age, education and head size. For each imaging endpoint, a mixed-effects model was defined to assess the relationship between effects of age, education, head size, APOE4/4 as covariates, and their interactions with time, in days from baseline scan. Likelihood ratio test was used to assess the significance of APOE4/4 × time interactions. Only APOE4/4 × time interaction was retained given the smaller sample size. All analyses were performed using the R package (R Foundation for Statistical Computing, Vienna, Austria. URL https://www.R-project.org). P-value < 0.05 was considered statistically significant, and p-value < 0.1 was considered a positive trend. All MRI analyses were performed by BioClinica (previously Synarc).

*Correlations of Clinical and Imaging Changes*

The cognitive scales included the cognitive subscale of the Alzheimer's Disease Assessment Scale with 13 items (ADAS-cog13) and the MMSE. The functional/composite scale was the Clinical Dementia Rating - Sum of Boxes (CDR-SB). These assessments were collected up to 7 times, at Baseline and Months 3, 6, 9, 12, 18 and 24. Details of the clinical assessments in ADNI-1 have been previously described (see Petersen cited above). For the MRI subgroup, baseline and change from baseline (CBL) clinical scores were analyzed. Clinical score changes were estimated by fitting a linear model for each subject. APOE4/4 effects on clinical scores was assessed at Baseline and Month 24 using a two-sample t-test, after adjusting for age and years of education. A mixed-effects model was defined to assess the relationship between effects of age, education, APOE4/4 and their interactions with time (in months from baseline visit). The likelihood ratio test was used to assess the significance of APOE4/4 × time interactions. Correlations between vMRI changes over 24 month and clinical change scores were analyzed by Pearson's correlations. Comparisons were focused on APOE3/3 and APOE4/4 subjects in the LMCI and AD groups.

*Tramiprosate MRI Dataset from the North American Phase 3 Trial*

The vMRI analyses came from a Phase 3 trial of oral tramiprosate in mild to moderate AD that included an MRI sub-study. See Gauthier S, Aisen PS, Ferris SH, Saumier D, Duong A, Haine A, et al. Effect of tramiprosate in patients with mild-to-moderate Alzheimer's disease: exploratory analyses of the MRI sub-group of the Alphase study. J Nutr Health & Aging 2009;13:550-57. We focused on the APOE4/4 group with mild AD (MMSE 20-26 inclusive), who had shown clinical benefit with tramiprosate. See Abushakra S, Porsteinsson A, Vellas B, Cummings J, Gauthier S, Hey JA, et al. Clinical benefits of tramiprosate in Alzheimer's disease are associated with higher number of APOE4 alleles: the "APOE4 gene-dose effect." J Prev Alz Dis 2016; 3(4):219-28 and Abushakra S, Porsteinsson A, Scheltens P, Sadowsky C, Vellas B, Cummings J, et al. Clinical Effects of Oral Tramiprosate in APOE4/4 Homozygous Patients with Mild Alzheimer's Disease Suggest Disease Modification. J Prev Alz Dis 2017;4 (3):149-56. The mild AD group with serial MRI included 28 APOE4/4 subjects (15 in placebo arm) and 62 APOE3/3 subjects (16 in placebo arm). Images were obtained using mostly 1.5 Tesla MRI at baseline and 18 months, and vMRI analysis methods were similar to those used for the ADNI-1 dataset. Tramiprosate vMRI analyses included HV and WBV.

**B. Results**

*Demographics and Baseline Characteristics*

Demographics and baseline scores of the ADNI-1 dataset of APOE4/4 and APOE3/3 subjects are shown in **Table** 2. This dataset includes LMCI and mild AD subjects with serial MRI. The ADNI APOE4/4 and APOE3/3 groups showed similar demographic and baseline clinical characteristics except for APOE4/4 group being significantly younger (approximately 5 years), and APOE4/4 group with LMCI had worse ADAS-cog13 scores than APOE3/3 subjects (~3 points, p = .016).

**Table 2**

Demographics and baseline characteristics of ADNI subjects with LMCI and mild AD

| Characteristics | LMCI<br>APOE3/3 N = 93 | LMCI<br>APOE4/4 N = 29 | LMCI<br>*P* value | Mild AD<br>APOE3/3 N = 29 | Mild AD<br>APOE4/4 N = 21 | Mild AD<br>*P* value |
|---|---|---|---|---|---|---|
| Age (years) | 75.96 (7.56) | 71.22 (5.88) | **.002** | 76.47 (8.79) | 71.63 (7.53) | **.047** |
| Gender (% male) | 67% | 62% | NA | 48% | 57% | |
| Education (years) | 15.86 (2.96) | 15.76 (2.40) | NS | 15.52 (3.31) | 14.52 (2.04) | NS |
| MMSE | 27.08 (1.84) | 26.69 (1.77) | NS | 23.34 (2.09) | 23.43 (1.86) | NS |
| ADAS-cog13 | 17.38 (5.90) | 20.39 (5.53) | **.016** | 29.39 (8.91) | 29.32 (6.08) | NS |
| CDR-SB | 1.45 (0.83) | 1.57 (0.87) | NS | 4.26 (1.49) | 4.57 (1.54) | NS |
| Hippocampus volume in cc | 6.26 (0.95) | 5.46 (0.73) | **< .001** | 5.95 (1.26) | 4.82 (0.76) | **< .001** |
| Whole brain volume in cc | 995.35 (56.12) | 975.02 (58.26) | .094 | 963.47 (59.08) | 933.54 (56.12) | .077 |

Abbreviations: LMCI, late mild cognitive impairment; ADNI, Alzheimer's Disease Neuroimaging Initiative; ADAS-cog13, the 13 item Alzheimer's Disease Assessment Scale-cognitive subscale; CDR-SB, Clinical Dementia Rating - Sum of Boxes; MMSE, Mini-Mental State Examination. APEO3/3 and APOE4/4 values are shown in mean (SD). Hippocampus volume and whole brain volume were adjusted for age, years of education, and head size. NS, not significant.

Demographics and baseline clinical scores of the tramiprosate APOE4/4 and APOE3/3 subjects with mild AD are shown in **Table 3.** The APOE4/4 group was also younger than APOE3/3 group by approximately 2 years, but this difference was not significant. The APOE4/4 mild AD groups from the 2 datasets showed similar demographic characteristics, except for higher % of men in the ADNI study. The tramiprosate study evaluated ADAS-cog11 rather than ADAS-cog13, but the 2 studies showed similar MMSE and CDR-SB scores in APOE4/4 subjects.

**Table 3**

Baseline characteristics of tramiprosate subjects with mild AD (MRI dataset)

| Characteristics | APOE3/3 N = 62 | APOE4/4 N = 28 | *P* value |
|---|---|---|---|
| Age (years) | 71.9 (9.8) | 70.1 (7.5) | NS |
| Gender (% male) | 55% | 43% | |
| Education (years) | 13.8 (3.6) | 13.7 (2.5) | NS |
| MMSE | 23.2 (1.89) | 23.3 (1.97) | NS |
| ADAS-cog11 | 17.9 (6.02) | 17.5 (5.24) | NS |
| CDR-SB | 4.8 (1.77) | 4.6 (1.84) | NS |
| Hippocampus volume in cc | 3.5 (0.79) | 3.1 (0.78) | **.026** |
| Whole brain volume in cc | 1074.4 (92.21) | 1076.3 (104.60) | NS |

Abbreviations: ADAS-cog11, the 11 item Alzheimer's Disease Assessment Scale-cognitive subscale; CDR-SB, Clinical Dementia Rating - Sum of Boxes; MMSE, Mini-Mental State Examination. APEO3/3 and APOE4/4 values are shown in mean (SD). Hippocampus volume and whole brain volume were adjusted for age, years of education, and head size. NS, not significant.

The ADNI APOE4/4 subjects had significantly smaller HV than APOE3/3 subjects (p< .001), with the smallest HV observed in APOE4/4 mild AD followed by APOE4/4 LMCI subjects. Similar to the ADNI data, the tramiprosate APOE4/4 group with mild AD had significantly smaller baseline HV compared to APOE3/3 subjects. In the tramiprosate dataset, HV was smaller, while WBV was slightly larger, compared to ADNI dataset.

*Baseline Volumetric MRI*

In the ADNI dataset, comparisons of baseline HV, cortical thickness and WBV between APOE3/3 and APOE4/4 groups are shown in **Table 4.** Baseline cortical thickness and WBV did not show significant differences between these 2 groups, while HV shows significantly smaller baseline values in APOE4/4 than APOE3/3 groups in both LMCI and mild AD.

**Table 4**

Atrophy rates of vMRI measures in LMCI & mild AD from ADNI dataset (APOE3/3 versus APOE4/4)

**Panel A: Rates in MCI**

| | **HV** | | | **Cortical Thickness** | | | **WBV** | | |
|---|---|---|---|---|---|---|---|---|---|
| **Timepoint** | **APOE3/3** | **APOE4/4** | ***P* value *** | **APOE3/3** | **APOE4/4** | ***P* value*** | **APOE3/3** | **APOE4/4** | ***P* value *** |
| N | 93 | 29 | | 93 | 29 | | 93 | 29 | |
| Baseline mean | 6387 (1083) | 5850 (845) | **.007** | 2.65 (0.16) | 2.62 (0.19) | .38 | 1022.6 (107.6) | 1020.6 (84.4) | .918 |
| **12 month** change | -2.76% (3.03) | -4.41% (2.62) | **.006** | -1.79% (1.60) | -3.13% (1.24) | < **.001** | -0.78% (0.98) | -1.08% (0.87) | .12 |
| **24 month** change | -6.17% (5.14) | -9.73% (4.67) | **.001** | -3.51% (2.54) | -6.03% (2.16) | < **.001** | -1.62% (1.35) | -2.37% (1.24) | **.008** |

**Panel B: Rates in mild AD**

| **Timepoint** | **HV** | | | **Cortical Thickness** | | | **WBV** | | |
|---|---|---|---|---|---|---|---|---|---|
| | **APOE3/3** | **APOE4/4** | ***P* value *** | **APOE3/3** | **APOE4/4** | ***P* value*** | **APOE3/3** | **APOE4/4** | ***P* value*** |
| N | 29 | 21 | | 29 | 21 | | 29 | 21 | |
| Baseline mean | 6039 (1447) | 5323 (890) | **.04** | 2.51 (0.24) | 2.50 (0.17) | .936 | 978.1 (103.8) | 997.0 (121.3) | .568 |
| **12 month** change | -5.43% (3.38) | -7.44% (3.73) | **.06†** | -3.28% (1.50) | -4.68% (2.28) | **.011** | -1.47% (1.00) | -1.53% (0.73) | .801 |
| **24 month** Change# | -10.74% (5.73) | -16.10% (6.60) | **.005** | -6.55% (3.00) | -8.62% (3.22) | **.025** | -2.73% (1.22) | -3.25% (1.09) | .122 |

Abbreviations: vMRI, volumetric magnetic resonance imaging; LMCI, late mild cognitive impairment; ADNI, Alzheimer's Disease Neuroimaging Initiative. HV, hippocampus volume; WBV, whole brain volume. APEO3/3 and APOE4/4 values are shown in mean (SD). # Number of APOE3/3 AD subjects at 24 months was 28. **P* value of *APOE3/3* vs. *APOE4*/4 < 0.05 is statistically significant, †*P* < 0.1 is a positive trend.

*Comparisons of vMRI Atrophy Rates over 12 and 24 Months in ADNI dataset*

In the LMCI group at 12 months, APOE4/4 homozygotes showed significantly higher % atrophy of HV and cortical thickness, but not WBV, compared to APOE3/3 subjects. At 24 months, all three vMRI measures showed significantly higher % atrophy in APOE4/4 compared to APOE3/3 subjects **(Table 4).**

In the mild AD group at 12 months, APOE4/4 homozygotes showed significantly higher % atrophy of cortical thickness, and a trend to higher % atrophy on HV (p< 0.1), compared to APOE3/3 subjects. At 24 months, both cortical

thickness and HV showed significantly higher % atrophy in APOE4/4 subjects **(Table 4).** In contrast, WBV atrophy rates in mild AD were not significantly different between APOE4/4 and APOE3/3 subjects at either 12 or 24 months. In APOE4/4 subjects, HV atrophy rates at 12 and 24 months in mild AD were higher than respective rates in LMCI group **(Table 4).**

When comparisons of atrophy rates between APOE4/4 and APOE3/3 subjects were repeated in the CSF positive subgroup, the results were similar. WBV did not show significant differences in atrophy between the two genotypes. In contrast, APOE4/4 subjects with LMCI and mild AD had significantly higher atrophy rates of cortical thickness, and their HV atrophy rates showed either a positive trend or were significantly higher than APOE3/3 subjects.

*Correlations of vMRI Rates of Change to Clinical Change Scores in APOE4/4 Homozygotes*

In the APOE4/4 subjects with LMCI, all vMRI changes were significantly correlated with the cognitive outcomes, with stronger correlations with ADAS-cog than MMSE. HV changes showed significant correlations to both ADAS-cog (r = -0.55, $P$ = .002) and MMSE (r = 0.39, $P$ = .037). Cortical thickness changes correlations with ADAS-cog (r = -0.59, $P$ < .001) and MMSE (r = 0.38, $P$ = .041) were also significant. The WBV change correlations were strong with both ADAS-cog (r = -0.64, $P$ < .0002) and MMSE (r = -0.64, $P$ < .0002). In contrast, vMRI changes were not significantly correlated with CDR-SB changes.

In the APOE4/4 with mild AD, the vMRI changes did not show significant correlations with clinical score changes.

*Annualized HV Atrophy Rates in APOE4/4 Homozygotes from ADNI-1 and Tramiprosate Mild AD groups*

Annualized HV atrophy rates were calculated to allow direct comparsion between tramiprosate data at 18 months and ADNI data at 12 and 24 months. The tramiprosate placebo group with serial MRIs included a total of 15 APOE4/4 subjects and 14 noncarrier subjects. Annualized HV atrophy rates were significantly higher in the APOE4/4 group (5.8%, SD = 5.0) than noncarrier group (3.4%, SD = 2.0) at 18 months. The APOE4/4 annualized atrophy rate in the tramiprosate mild AD group was not significantly different from the ADNI LMCI group at 12 and 24 months (4.4%, SD 2.6; 4.9%, SD = 2.3), or mild AD group at 12 and 24 months (7.4%, SD 3.7; 8.1%, SD = 3.3). Comparison of tramiprosate and ADNI mild AD atrophy rate at 24 month showed p= 0.1, all others were > 0.1 data shown in **FIG. 1.**

**C. Discussion**

The analyses of two independent clinical datasets above evidence that APOE4/4 homozygotes represent a distinct biological and clinical phenotype of AD with clear differences from APOE3/3 subjects. APOE4/4 homozygotes were significantly younger than APOE3/3 subjects, yet had significantly smaller HV. LMCI subjects also had significantly worse baseline cognitive scores. This is consistent with metabolic imaging (FDG-PET) studies showing early metabolic dysfunction in *APOE4* carriers with MCI, compared to noncarriers. See e.g., Scarmeas N, Habeck C, Anderson KE, Hilton J, Devanand DP, Pelton GH, et al. Altered PET functional brain responses in cognitively intact elderly persons at risk for Alzheimer disease (carriers of the ε4 allele). Am J Geriatr Psychiatry 2004;12:596-605; Mosconi L, Nacmias B, Sorbi S, De Cristofaro MTR, Fayazz M, Tedde A, et al. Brain metabolic decreases related to the dose of the APOE e4 allele in Alzheimer's disease. J Neurol Neurosurg Psychiatry 2004;75:370-376; Knopman DS, Jack CR Jr, Wiste HJ, Lundt ES, Weigand SD, Vemuri P, et al. 18F-fluorodeoxyglucose positron emission tomography, aging, and apolipoprotein E genotype in cognitively normal persons. Neurobiol Aging 2014;35:2096-106; and Paranjpe M, Chen X, Liu M, Paranjpe I, Leal JP, Wanget R, et al. The effect of APOE ε4 on longitudinal brain region-specific glucose metabolism in patients with mild cognitive impairment: a FDG-PET study. Neuroimage Clin. 2019; 22:101795.

APOE4/4 homozygotes also showed accelerated hippocampus atrophy compared to APOE3/3 subjects at both the LMCI and mild AD stages. The HV atrophy rates were higher in mild AD than LMCI, with annualized rates in LMCI at 24 months of ~ 5%, and in mild AD of ~8%. HV atrophy in LMCI correlated with cognitive decline.

APOE4/4 subjects with early AD subjects also showed accelerated rates of cortical thickness loss, compared to APOE3/3 subjects, at both the MCI and mild AD stages. Cortical thickness showed a similar profile to HV, with significantly higher atrophy rates in APOE4/4 homozygotes than APOE3/3 subjects, at 12 and 24 months. In contrast, WBV did not show consistent differences between the genotypes.

Yet another finding shown above was that changes in ADAS-cog, but not CDR-SB, correlated well with HV and cortical thickness changes in LMCI. This finding is consistent with observations from early clinical trials, where APOE4 carriers with early/mild AD showed a larger efficacy signal on ADAS-cog than CDR-SB. See Cummings J. Clinical and Biomarker Updates from BAN2401 Study 201 in Early AD. Symposium at Clinical Trials in Alzheimer's Disease, October 2018 and Abushakra cited above.

The above findings support hippocampus volume and cortical thickness as markers for treatment and drug efficacy evaluation in APOE4/4 homozygotes.

While we have described a number of embodiments of this invention, it is apparent that our basic examples may

be altered to provide other embodiments that utilize the compounds and methods of this invention. Therefore, it will be appreciated that the scope of this invention is to be defined by the appended claims rather than by the specific embodiments that have been represented by way of example.

## Claims

**1.** A compound for use in treating Alzheimer's disease, wherein the treatment comprises

a. selecting an adult human subject who is determined to:

i. comprise an APOE4/APOE4, APOE3/APOE4, or APOE2/APOE4 genotype,
ii. be asymptomatic for Alzheimer's disease, and
iii. have at least one of:

(1) a hippocampal volume below a threshold level; or
(2) cortical thickness below a threshold level; and

b. administering to the selected subject a therapeutically effective amount of the compound,
wherein the compound is tramiprosate, a pharmaceutically acceptable salt of tramiprosate, a tramiprosate prodrug, a tramiprosate derivative, or an active tramiprosate metabolite;
wherein for subjects comprising an APOE4/APOE4 genotype, the threshold levels are

- the hippocampal volume is at least 5% lower than a baseline hippocampal volume obtained within 12 months prior to selection,
- the hippocampal volume is at least 10% lower than a baseline hippocampal volume obtained less than 24 months prior to selection,
- the hippocampal volume is less than 4.75 $cm^3$,
- the cortical thickness is at least 3% less than a baseline cortical thickness obtained within 12 months prior to selection,
- the cortical thickness is at least 6% less than a baseline cortical thickness obtained within 24 months prior to selection, or
- the cortical thickness is less than 2.50 mm;

wherein for subjects comprising an APOE3/APOE4 genotype, the subject is selected only if the subject is further determined to be positive for amyloid deposits in the brain, and the threshold levels are

- the hippocampal volume is at least 5% lower than a baseline hippocampal volume obtained within 12 months prior to the selection,
- the hippocampal volume is at least 10% lower than a baseline hippocampal volume obtained less than 24 months prior to the selection,
- the hippocampal volume is less than 5.10 $cm^3$,
- the cortical thickness is at least 3% less than a baseline cortical thickness obtained within 12 months prior to the selection,
- if the cortical thickness is at least 6% less than a baseline cortical thickness obtained within 24 months prior to the selection, or
- the cortical thickness is less than 2.50 mm;

wherein for subjects comprising an APOE2/APOE4 genotype, the subject is selected only if the subject is further determined to be positive for amyloid deposits in the brain, and the threshold levels are

- the hippocampal volume is at least 4% lower than a baseline hippocampal volume obtained within 12 months prior to the selection,
- the hippocampal volume is at least 7% lower than a baseline hippocampal volume obtained less than 36 months prior to the selection,
- the hippocampal volume is less than 6.10 $cm^3$,
- cortical thickness is at least 2% less than a baseline cortical thickness obtained within 12 months prior to the selection,

- the subject is selected if the cortical thickness is at least 4% less than a baseline cortical thickness obtained within 36 months prior to the selection, or
- the cortical thickness is less than 2.50 mm.

**2.** A compound for use in treating Alzheimer's disease in a subject, wherein the treatment comprises administering to the subject a therapeutically effective amount of the compound,
wherein the compound is tramiprosate, a pharmaceutically acceptable salt of tramiprosate, a tramiprosate prodrug, a tramiprosate derivative, or an active tramiprosate metabolite; and

wherein the subject is an adult human who:

i. comprises an APOE4/APOE4, APOE3/APOE4, or APOE2/APOE4 genotype,
ii. is asymptomatic for Alzheimer's disease, and
iii. has at least one of:

(1) a hippocampal volume below a threshold level; or
(2) cortical thickness below a threshold level; and

wherein for subjects comprising an APOE4/APOE4 genotype, the threshold levels are

- the hippocampal volume is at least 5% lower than a baseline hippocampal volume obtained within 12 months prior to selection,
- the hippocampal volume is at least 10% lower than a baseline hippocampal volume obtained less than 24 months prior to selection,
- the hippocampal volume is less than 4.75 $cm^3$,
- the cortical thickness is at least 3% less than a baseline cortical thickness obtained within 12 months prior to selection,
- the cortical thickness is at least 6% less than a baseline cortical thickness obtained within 24 months prior to selection, or
- the cortical thickness is less than 2.50 mm;

wherein for subjects comprising an APOE3/4 genotype, the subject is positive for amyloid deposits in the brain, and the threshold levels are

- the hippocampal volume is at least 5% lower than a baseline hippocampal volume obtained within 12 months prior to the selection,
- the hippocampal volume is at least 10% lower than a baseline hippocampal volume obtained less than 24 months prior to the selection,
- the hippocampal volume is less than 5.10 $cm^3$,
- the cortical thickness is at least 3% less than a baseline cortical thickness obtained within 12 months prior to the selection,
- if the cortical thickness is at least 6% less than a baseline cortical thickness obtained within 24 months prior to the selection, or
- the cortical thickness is less than 2.50 mm;

wherein for subjects comprising an APOE2/APOE4 genotype, the subject is positive for amyloid deposits in the brain, and the threshold levels are

- the hippocampal volume is at least 4% lower than a baseline hippocampal volume obtained within 12 months prior to the selection,
- the hippocampal volume is at least 7% lower than a baseline hippocampal volume obtained less than 36 months prior to the selection,
- the hippocampal volume is less than 6.10 $cm^3$,
- cortical thickness is at least 2% less than a baseline cortical thickness obtained within 12 months prior to the selection,
- the subject is selected if the cortical thickness is at least 4% less than a baseline cortical thickness obtained within 36 months prior to the selection, or
- the cortical thickness is less than 2.50 mm.

3. The compound for use according to any of Claims 1 to 2, wherein the compound is a tramiprosate prodrug of the formula:

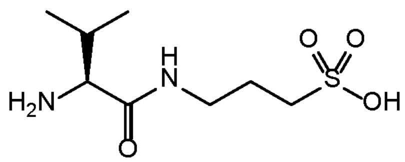

,

or a pharmaceutically acceptable salt thereof.

4. The compound for use according to any one of Claims 1 to 3, wherein the compound is a tramiprosate metabolite of the formula:

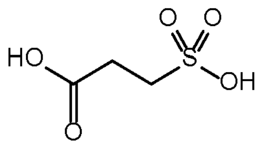

,

or a pharmaceutically acceptable salt thereof.

5. The compound for use according to any one of the preceding claims, wherein the subject is determined to be asymptomatic for Alzheimer's disease if the subject scores 28 or higher on a MMSE.

6. The compound for use according to any one of the preceding claims, wherein one or both of hippocampal volume and cortical thickness is measured by MRI.

7. The compound for use according to any one of the preceding claims, wherein amyloid deposits are detected by PET imaging or cerebral spinal fluid biomarkers.

**Patentansprüche**

1. Verbindung, die zur Behandlung der Alzheimer-Krankheit verwendet wird, wobei die Behandlung Folgendes umfasst:

a. Auswählen eines erwachsenen Probanden, der entschlossen ist:

i. einen APOE4/APOE4-, APOE3/APOE4- oder APOE2/APOE4-Genotyp zu umfassen,
ii. symptomfrei für Alzheimer zu sein und
iii. mindestens eines der folgenden Kriterien zu erfüllen:

(1) ein Hippocampusvolumen unterhalb eines Schwellenwerts; oder
(2) eine kortikale Dicke unterhalb eines Schwellenwerts; und

b. Verabreichen einer therapeutisch wirksamen Menge der Verbindung an den ausgewählten Probanden,

wobei die Verbindung Tramiprosat, ein pharmazeutisch verträgliches Salz von Tramiprosat, ein Tramiprosat-Prodrug, ein Tramiprosat-Derivat oder ein aktiver Tramiprosat-Metabolit ist;
wobei für Probanden umfassend, einen APOE4/APOE4-Genotyp, die Schwellenwerte wie folgt sind

- das Hippocampusvolumen ist mindestens 5 % geringer als das Basis-Hippocampusvolumen, das innerhalb von 12 Monaten vor der Auswahl ermittelt wurde,
- das Hippocampusvolumen ist mindestens 10 % geringer als das Basis-Hippocampusvolumen, das weniger als 24 Monate vor der Auswahl ermittelt wurde,
- das Hippocampusvolumen ist weniger als 4,75 $cm^3$,
- die kortikale Dicke ist mindestens 3 % geringer als die innerhalb von 12 Monaten vor der Auswahl

ermittelte kortikale Basisdicke,
- die kortikale Dicke ist mindestens 6 % geringer als die innerhalb von 24 Monaten vor der Auswahl ermittelte kortikale Basisdicke oder
- die kortikale Dicke ist weniger als 2,50 mm;

wobei für Probanden umfassend, einen APOE3/APOE4-Genotyp, der Proband nur ausgewählt wird, wenn bei ihm zusätzlich Amyloidablagerungen im Gehirn festgestellt werden, und die Schwellenwerte wie folgt sind

- das Hippocampusvolumen ist mindestens 5 % geringer als das Basis-Hippocampusvolumen, das innerhalb von 12 Monaten vor der Auswahl ermittelt wurde,
- das Hippocampusvolumen ist mindestens 10 % geringer als das Basis-Hippocampusvolumen, das weniger als 24 Monate vor der Auswahl ermittelt wurde,
- das Hippocampusvolumen ist weniger als 5,10 $cm^3$,
- die kortikale Dicke ist mindestens 3 % geringer als die innerhalb von 12 Monaten vor der Auswahl ermittelte kortikale Basisdicke,
- wenn die kortikale Dicke mindestens 6 % geringer ist als die innerhalb von 24 Monaten vor der Auswahl ermittelte kortikale Basisdicke oder
- die kortikale Dicke ist weniger als 2,50 mm;

wobei für Probanden umfassend, einen APOE2/APOE4-Genotyp, der Proband nur ausgewählt wird, wenn bei ihm zusätzlich Amyloidablagerungen im Gehirn festgestellt werden, und die Schwellenwerte wie folgt sind

- das Hippocampusvolumen ist mindestens 4 % geringer als das Basis-Hippocampusvolumen, das innerhalb von 12 Monaten vor der Auswahl ermittelt wurde,
- das Hippocampusvolumen ist mindestens 7 % geringer als das Basis-Hippocampusvolumen, das weniger als 36 Monate vor der Auswahl ermittelt wurde,
- das Hippocampusvolumen ist weniger als 6,10 $cm^3$,
- die kortikale Dicke ist mindestens 2 % geringer als die innerhalb von 12 Monaten vor der Auswahl ermittelte kortikale Basisdicke.
- der Proband wird ausgewählt, wenn die kortikale Dicke mindestens 4 % geringer ist als die innerhalb von 36 Monaten vor der Auswahl ermittelte kortikale Basisdicke oder
- die kortikale Dicke ist weniger als 2,50 mm.

**2.** Verbindung, die zur Behandlung der Alzheimer-Krankheit bei einem Patienten verwendet wird, wobei die Behandlung die Verabreichung einer therapeutisch wirksamen Menge der Verbindung an den Patienten umfasst. wobei die Verbindung Tramiprosat, ein pharmazeutisch verträgliches Salz von Tramiprosat, ein Tramiprosat-Prodrug, ein Tramiprosat-Derivat oder ein aktiver Tramiprosat-Metabolit ist; und

wobei es sich bei dem Probanden um einen erwachsenen Menschen handelt, der:

i. einen APOE4/APOE4-, APOE3/APOE4- oder APOE2/APOE4-Genotyp umfasst,
ii. symptomfrei für Alzheimer ist und
iii. mindestens eines der folgenden Kriterien erfüllt:

(1) ein Hippocampusvolumen unterhalb eines Schwellenwerts; oder
(2) eine kortikale Dicke unterhalb eines Schwellenwerts; und

wobei für Probanden umfassend, einen APOE4/APOE4-Genotyp, die Schwellenwerte wie folgt sind

- das Hippocampusvolumen ist mindestens 5 % geringer als das Basis-Hippocampusvolumen, das innerhalb von 12 Monaten vor der Auswahl ermittelt wurde,
- das Hippocampusvolumen ist mindestens 10 % geringer als das Basis-Hippocampusvolumen, das weniger als 24 Monate vor der Auswahl ermittelt wurde,
- das Hippocampusvolumen ist weniger als 4,75 $cm^3$,
- die kortikale Dicke ist mindestens 3 % geringer als die innerhalb von 12 Monaten vor der Auswahl ermittelte kortikale Basisdicke,

- die kortikale Dicke ist mindestens 6 % geringer als die innerhalb von 24 Monaten vor der Auswahl ermittelte kortikale Basisdicke oder
- die kortikale Dicke ist weniger als 2,50 mm;

wobei für Probanden umfassend, einen APOE3/4-Genotyp, der Proband positiv auf Amyloidablagerungen im Gehirn getestet wurden und die Schwellenwerte wie folgt sind

- das Hippocampusvolumen ist mindestens 5 % geringer als das Basis-Hippocampusvolumen, das innerhalb von 12 Monaten vor der Auswahl ermittelt wurde,
- das Hippocampusvolumen ist mindestens 10 % geringer als das Basis-Hippocampusvolumen, das weniger als 24 Monate vor der Auswahl ermittelt wurde,
- das Hippocampusvolumen ist weniger als 5,10 $cm^3$,
- die kortikale Dicke ist mindestens 3 % geringer als die innerhalb von 12 Monaten vor der Auswahl ermittelte kortikale Basisdicke,
- wenn die kortikale Dicke mindestens 6 % geringer ist als die innerhalb von 24 Monaten vor der Auswahl ermittelte kortikale Basisdicke oder
- die kortikale Dicke ist weniger als 2,50 mm;

wobei für Probanden umfassend, einen APOE2/APOE4-Genotyp, der Proband positiv auf Amyloidablagerungen im Gehirn getestet wurden und die Schwellenwerte wie folgt sind

- das Hippocampusvolumen ist mindestens 4 % geringer als das Basis-Hippocampusvolumen, das innerhalb von 12 Monaten vor der Auswahl ermittelt wurde,
- das Hippocampusvolumen ist mindestens 7 % geringer als das Basis-Hippocampusvolumen, das weniger als 36 Monate vor der Auswahl ermittelt wurde,
- das Hippocampusvolumen ist weniger als 6,10 $cm^3$,
- die kortikale Dicke ist mindestens 2 % geringer als die innerhalb von 12 Monaten vor der Auswahl ermittelte kortikale Basisdicke,
- der Proband wird ausgewählt, wenn die kortikale Dicke mindestens 4 % geringer ist als die innerhalb von 36 Monaten vor der Auswahl ermittelte kortikale Basisdicke oder
- die kortikale Dicke ist weniger als 2,50 mm.

**3.** Verbindung zur Verwendung nach einem der Ansprüche 1 bis 2, wobei die Verbindung

ein Tramiprosat-Prodrug der folgenden Formel ist:

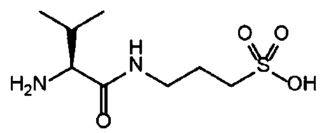

,

oder ein
pharmazeutisch verträgliches Salz davon.

**4.** Verbindung zur Verwendung nach einem der Ansprüche 1 bis 3, wobei die Verbindung ein Tramiprosat-Metabolit der folgenden Formel ist:

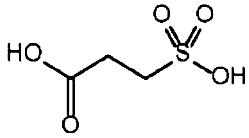

,

oder ein pharmazeutisch verträgliches Salz davon.

**5.** Verbindung, zur Verwendung nach einem der vorstehenden Ansprüche, wobei der Proband als symptomfrei für die Alzheimer-Krankheit eingestuft wird, wenn er bei einem MMSE-Test 28 oder mehr Punkte erzielt.

6. Verbindung zur Verwendung nach einem der vorstehenden Ansprüche, wobei das Hippocampusvolumen und/oder die kortikale Dicke mittels MRT gemessen wird.

7. Verbindung zur Verwendung nach einem der vorstehenden Ansprüche, wobei Amyloidablagerungen durch PET-Bildgebung oder Biomarker im Liquor cerebrospinalis nachgewiesen werden.

## Revendications

1. Composé destiné au traitement de la maladie d'Alzheimer, dans lequel le traitement comprend

a. la sélection d'un sujet humain adulte déterminé à :

i. comprendre un génotype APOE4/APOE4, APOE3/APOE4 ou APOE2/APOE4,
ii. être asymptomatique pour la maladie d'Alzheimer, et
iii. posséder au moins l'un :

(1) d'un volume hippocampique inférieur à un seuil ; ou
(2) d'une épaisseur corticale inférieure à un seuil ; et

b. l'administration au sujet sélectionné d'une quantité thérapeutiquement efficace du composé,
dans lequel le composé est le tramiprosate, un sel pharmaceutiquement acceptable du tramiprosate, une prodrogue du tramiprosate, un dérivé du tramiprosate ou un métabolite actif du tramiprosate ;
dans lequel, pour les sujets présentant un génotype APOE4/APOE4, les seuils sont

- le volume de l'hippocampe est inférieur d'au moins 5% au volume de l'hippocampe de référence obtenu dans les 12 mois précédant la sélection,
- le volume de l'hippocampe est inférieur d'au moins 10% au volume de l'hippocampe de référence obtenu moins de 24 mois avant la sélection,
- le volume de l'hippocampe est inférieur à 4,75 $cm^3$,
- l'épaisseur corticale est inférieure d'au moins 3% à l'épaisseur corticale de référence obtenue dans les 12 mois précédant la sélection,
- l'épaisseur corticale est inférieure d'au moins 6% à l'épaisseur corticale de référence obtenue dans les 24 mois précédant la sélection, ou
- l'épaisseur corticale est inférieure à 2,50 mm ; dans lequel, pour les sujets présentant un génotype APOE3/APOE4, le sujet n'est sélectionné que s'il est par ailleurs confirmé positif pour les dépôts amyloïdes dans le cerveau, et les seuils sont
- le volume de l'hippocampe est inférieur d'au moins 5% au volume de l'hippocampe de référence obtenu dans les 12 mois précédant la sélection,
- le volume de l'hippocampe est inférieur d'au moins 10% au volume de l'hippocampe de référence obtenu moins de 24 mois avant la sélection,
- le volume de l'hippocampe est inférieur à 5,10 $cm^3$,
- l'épaisseur corticale est inférieure d'au moins 3% à l'épaisseur corticale de référence obtenue dans les 12 mois précédant la sélection,
- si l'épaisseur corticale est inférieure d'au moins 6% à l'épaisseur corticale de référence obtenue dans les 24 mois précédant la sélection, ou
- l'épaisseur corticale est inférieure à 2,50 mm ; dans lequel, pour les sujets présentant un génotype APOE2/APOE4, le sujet n'est sélectionné que s'il est par ailleurs confirmé positif pour les dépôts amyloïdes dans le cerveau, et les seuils sont
- le volume de l'hippocampe est inférieur d'au moins 4% au volume de l'hippocampe de référence obtenu dans les 12 mois précédant la sélection,
- le volume de l'hippocampe est inférieur d'au moins 7% au volume de l'hippocampe de référence obtenu moins de 36 mois avant la sélection,
- le volume de l'hippocampe est inférieur à 6,10 $cm^3$,
- l'épaisseur corticale est inférieure d'au moins 2% à l'épaisseur corticale de référence obtenu dans les 12 mois précédant la sélection,
- le sujet est sélectionné si l'épaisseur corticale est inférieure d'au moins 4% à l'épaisseur corticale de référence obtenue dans les 36 mois précédant la sélection, ou

- l'épaisseur corticale est inférieure à 2,50 mm.

2. Composé destiné au traitement de la maladie d'Alzheimer chez un sujet, dans lequel le traitement comprend l'administration au sujet d'une quantité thérapeutiquement efficace du composé,

dans lequel le composé est le tramiprosate, un sel pharmaceutiquement acceptable du tramiprosate, une prodrogue du tramiprosate, un dérivé du tramiprosate ou un métabolite actif du tramiprosate ; et
dans lequel le sujet est un être humain adulte qui :

i. comprend un génotype APOE4/APOE4, APOE3/APOE4 ou APOE2/APOE4,
ii. est asymptomatique pour la maladie d'Alzheimer, et
iii. possède au moins l'un :

(1) d'un volume hippocampique inférieur à un seuil donné ; ou
(2) d'une épaisseur corticale inférieure à un seuil ; et

dans lequel, pour les sujets présentant un génotype APOE4/APOE4, les seuils sont

- le volume de l'hippocampe est inférieur d'au moins 5% au volume de l'hippocampe de référence obtenu dans les 12 mois précédant la sélection,
- le volume de l'hippocampe est inférieur d'au moins 10% au volume de l'hippocampe de référence obtenu moins de 24 mois avant la sélection,
- le volume de l'hippocampe est inférieur à 4,75 $cm^3$,
- l'épaisseur corticale est inférieure d'au moins 3% à l'épaisseur corticale de référence obtenue dans les 12 mois précédant la sélection,
- l'épaisseur corticale est inférieure d'au moins 6% à l'épaisseur corticale de référence obtenue dans les 24 mois précédant la sélection, ou
- l'épaisseur corticale est inférieure à 2,50 mm ; dans lequel, pour les sujets présentant un génotype APOE3/4, le sujet est positif pour les dépôts amyloïdes dans le cerveau, et les niveaux seuils sont
- le volume de l'hippocampe est inférieur d'au moins 5% au volume de l'hippocampe de référence obtenu dans les 12 mois précédant la sélection,
- le volume de l'hippocampe est inférieur d'au moins 10% au volume de l'hippocampe de référence obtenu moins de 24 mois avant la sélection,
- le volume de l'hippocampe est inférieur à 5,10 $cm^3$,
- l'épaisseur corticale est inférieure d'au moins 3% à l'épaisseur corticale de référence obtenue dans les 12 mois précédant la sélection,
- si l'épaisseur corticale est inférieure d'au moins 6% à l'épaisseur corticale de référence obtenue dans les 24 mois précédant la sélection, ou
- l'épaisseur corticale est inférieure à 2,50 mm ; dans lequel, pour les sujets présentant un génotype APOE2/APOE4, le sujet est positif pour les dépôts amyloïdes dans le cerveau, et les niveaux seuils sont
- le volume de l'hippocampe est inférieur d'au moins 4% au volume de l'hippocampe de référence obtenu dans les 12 mois précédant la sélection,
- le volume de l'hippocampe est inférieur d'au moins 7% au volume de l'hippocampe de référence obtenu moins de 36 mois avant la sélection,
- le volume de l'hippocampe est inférieur à 6,10 $cm^3$,
- l'épaisseur corticale est inférieure d'au moins 2% à l'épaisseur corticale de référence obtenue dans les 12 mois précédant la sélection,
- le sujet est sélectionné si l'épaisseur corticale est inférieure d'au moins 4% à l'épaisseur corticale de référence obtenue dans les 36 mois précédant la sélection, ou
- l'épaisseur corticale est inférieure à 2,50 mm.

3. Composé destiné à être utilisé selon l'une quelconque des revendications 1 et 2, dans lequel le composé

est une prodrogue du tramiprosate de formule :

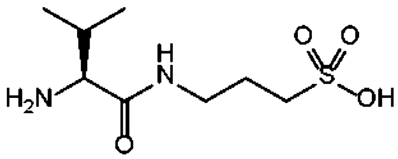

,

ou un sel pharmaceutiquement acceptable de celui-ci.

**4.** Composé destiné à être utilisé selon l'une quelconque des revendications 1 à 3, dans lequel le composé est un métabolite du tramiprosate de formule :

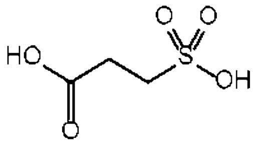

,

ou un sel pharmaceutiquement acceptable de celui-ci.

**5.** Composé destiné à être utilisé selon l'une quelconque des revendications précédentes, dans lequel le sujet est considéré comme asymptomatique pour la maladie d'Alzheimer si le sujet obtient un score de 28 ou plus au MMSE.

**6.** Composé destiné à être utilisé selon l'une quelconque des revendications précédentes, dans lequel le volume de l'hippocampe et/ou l'épaisseur corticale sont mesurés par IRM.

**7.** Composé destiné à être utilisé selon l'une quelconque des revendications précédentes, dans lequel les dépôts amyloïdes sont détectés par imagerie TEP ou par biomarqueurs du liquide céphalo-rachidien.

FIG. 1

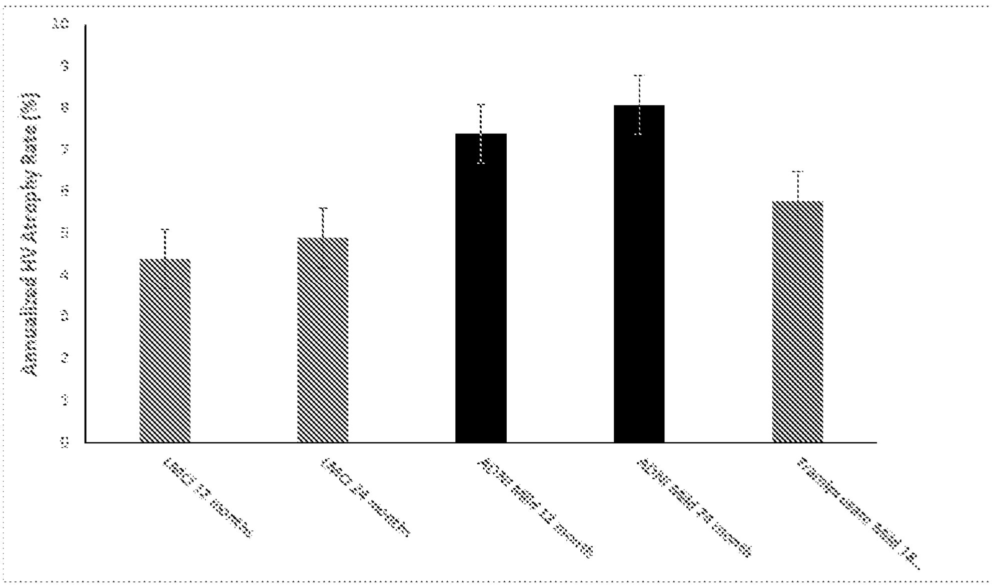


Abbreviations: vMRI, volumetric magnetic resonance imaging; LMCI, late mild cognitive impairment; error bars are standard errors of the mean. Number of subjects: LMCI, 29, Mild AD 21, tramiprosate Mild AD 15.*P values for Tramiprosate versus ADNI comparisons were not significant (all p values > 0.05).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015143447 A
- WO 2017044840 A
- US 62713056 B


### Non-patent literature cited in the description


- **VEITCH DP** ; **WEINER MW** ; **AISEN PS** ; **BECKETT LA** ; **CAIRNS NJ** ; **GREEN RC et al.** Understanding disease progression and improving Alzheimer's disease clinical trials: Recent highlights from the Alzheimer's Disease Neuroimaging Initiative. *Alzheimers Dement*, 2019, vol. 15, 106-52
- **CORDER EH** ; **SAUNDERS AM** ; **STRITTMATTER WJ** ; **SCHMECHEL DE** ; **GASKELL PC** ; **SMALL GW et al.** Gene dose of apolipoprotein E type 4 allele and the risk of Alzheimer's disease in late onset families. *Science*, 1993, vol. 261, 921-3
- *Alzheimer's disease. Annu Rev Med*, 1996, vol. 47, 387-400
- **REIMAN EM et al.** *PNAS*, 2009, vol. 106, 6820-25
- **JANSEN et al.** *JAMA*, 2015, vol. 313, 1924-38
- **OSSENKOPPELE R et al.** *JAMA*, 2015, vol. 313, 1939-49
- **DEGENHARDT EK et al.** *Psychosomatics*, 2016, vol. 57, 208-216
- **HASHIMOTO T et al.** *J Neurosci*, 2012, vol. 32, 15181-92
- **TAI LM** ; **BILOUSOVA T et al.** *J Biol Chem*, 2013, vol. 288 (8), 5914-26
- **VIOLA KL et al.** *Acta Neuropathol*, 2015, vol. 129, 183-206
- **HONG W et al.** *Acta Neuropathol*, 2018, vol. 136, 19-40
- **APOSTOLOVA LG et al.** *Neurobiol Aging*, 2010, vol. 31, 1284-1303
- **REIMAN EM et al.** *PNAS*, 2001, vol. 98, 3334-39
- **SCARMEAS N et al.** *Am J Geriatr Psychiatry*, 2004, vol. 12, 596-605
- **CASELLI RJ et al.** *N Engl J Med*, 2009, vol. 361, 255-63
- **ESPARZA TJ et al.** *Ann Neurol*, 2013, vol. 73, 104-19
- **KOCIS P et al.** *CNS Drugs*, 2017, vol. 31, 495-509
- **HEY JA et al.** *CNS Drugs*, 2018, vol. 32, 849-861
- **ABUSHAKRA S et al.** *J Prev Alz Dis*, 2016, vol. 3 (4), 219-28
- **GAUTHIER, S et al.** *J Nutr Health Aging*, 2009, vol. 13, 550-557
- **S. ABUSHAKRA et al.** *J Prev Alz Dis*, 2016, vol. 3 (4), 219-28
- **HEY et al.** *Clin Pharmacokinetics*, 2018, 315-333
- **KOCIS et al.** *CNS Drugs*, 2017, vol. 31, 495-509
- **ABUSHAKRA et al.** *J Prev Alzheimers Dis*, 2017, vol. 4, 149-56
- **D MEHTA et al.** *Expert Opin Investig Drugs*, 2017, vol. 26 (6), 735-739
- **GAUTHIER, S et al.** Effect of tramiprosate in patients with mild-to-moderate Alzheimer's disease: exploratory analyses of the MRI sub-group of the Alphase study. *J Nutr Health Aging*, 2009, vol. 13, 550-557
- **SAUMIER, D** ; **DUONG, A** ; **HAINE, D** ; **GARCEAU, D** ; **SAMPALIS, J**. Domain-specific cognitive effects of tramiprosate in patients with mild to moderate Alzheimer's disease: ADAS-cog subscale results from the Alphase Study. *J Nutr Health Aging*, 2009, vol. 13, 808-812
- **AISEN, P. S. et al.** Tramiprosate in mild-to-moderate Alzheimer's disease - a randomized, double-blind, placebo-controlled, multi-centre study (the Alphase Study). *Arch Med Sci*, 2011, vol. 7, 102-111
- **DUFF et al.** *Archives of Clinical Neuropsychology*, 2008, vol. 23, 603-612
- *Clin Neuropsychol*, August 2003, vol. 17 (3), 351-366
- **GRAHAM et al.** *Alzheimer Dis Assoc Disord*, 2004, vol. 18, 236-240
- **NABERS** ; **FORLENZA et al.** EMBO Molecular Medicine e8763/2018. *Alzheimer's and Dementia: Diagnosis, Assessment and Disease Monitoring*, 2015, vol. 1, 455-463
- Hope remains on Alzheimer's disease treatments: our in-depth look at biopharma's clinical pipeline. *Biotechnology industry*, 28 June 2019

- **SUMANT KULKARNI et al.** *Canaccord Genuity Capital Markets*
- **TK KARIKARI et al.** *Lancet Neurol*, 2020, vol. 19, 422-33
- **S JANELIDZE et al.** *Nature Med*, 2020, vol. 26, 379-86
- **NICOLAS R. BARTHÉLEMY et al.** *Alzheimer's Research & Therapy*, 2020, vol. 12 (26)
- **NJ ASHTON et al.** *Acta Neuropath Comm*, 2019, vol. 7 (5)
- **N MATTSSON et al.** *JAMA Neurol*, 2017, vol. 74 (5), 557-66
- **JACK CR JR** ; **BENNETT DA** ; **BLENNOW K** ; **CARRILLO MC** ; **DUNN B** ; **HAEBERLEIN SB et al.** NIA-AA Research Framework: Toward a biological definition of Alzheimer's disease. *Alzheimers Dement*, 2018, vol. 14, 535-562
- **PETERSEN RC** ; **AISEN PS** ; **BECKETT LA** ; **DONOHUE MC** ; **GAMST AC** ; **HARVEY DJ et al.** Alzheimer's Disease Neuroimaging Initiative (ADNI): clinical characterization. *Neurology*, 2010, vol. 74, 201-9
- **FISCHL B** ; **SALAT DH** ; **BUSA E** ; **ALBERT M** ; **DIETERICH M** ; **HASELGROVE C et al.** Whole brain segmentation: automated labeling of neuroanatomical structures in the human brain. *Neuron*, 2002, vol. 33, 341-55
- **FISCHL B** ; **VAN DER KOUWE A** ; **DESTRIEUX C** ; **HALGREN E** ; **SÉGONNE F** ; **SALAT DH et al.** Automatically parcellating the human cerebral cortex. *Cereb Cortex*, 2004, vol. 14, 11-22
- **LEUNG KK** ; **RIDGWAY GR** ; **OURSELIN S** ; **FOX NC**. Consistent multi-time-point brain atrophy estimation from the boundary shift interval. *NeuroImage*, 2012, vol. 59, 3995-4005
- **JACK CR JR** ; **WISTE HJ** ; **WEIGAND SD** ; **THERNEAU TM** ; **LOWE VJ** ; **KNOPMAN DS et al.** Defining imaging biomarker cut-points for brain aging and Alzheimer's disease. *Alzheimers Dement*, 2017, vol. 13, 205-216
- **SCHWARZ CG** ; **GUNTER JL** ; **WISTE HJ** ; **PRZYBELSKI SA** ; **WEIGAND SD** ; **WARD CP et al.** A large-scale comparison of cortical thickness and volume methods for measuring Alzheimer's Disease Severity. *Neuroimage*, 2016, vol. 11, 802-81
- **GAUTHIER S** ; **AISEN PS** ; **FERRIS SH** ; **SAUMIER D** ; **DUONG A** ; **HAINE A et al.** Effect of tramiprosate in patients with mild-to-moderate Alzheimer's disease: exploratory analyses of the MRI sub-group of the Alphase study. *J Nutr Health & Aging*, 2009, vol. 13, 550-57
- **ABUSHAKRA S** ; **PORSTEINSSON A** ; **VELLAS B** ; **CUMMINGS J** ; **GAUTHIER S** ; **HEY JA et al.** Clinical benefits of tramiprosate in Alzheimer's disease are associated with higher number of APOE4 alleles: the ''APOE4 gene-dose effect. *J Prev Alz Dis*, 2016, vol. 3 (4), 219-28
- **ABUSHAKRA S** ; **PORSTEINSSON A** ; **SCHELTENS P** ; **SADOWSKY C** ; **VELLAS B** ; **CUMMINGS J et al.** Clinical Effects of Oral Tramiprosate in APOE4/4 Homozygous Patients with Mild Alzheimer's Disease Suggest Disease Modification. *J Prev Alz Dis*, 2017, vol. 4 (3), 149-56
- **SCARMEAS N** ; **HABECK C** ; **ANDERSON KE** ; **HILTON J** ; **DEVANAND DP** ; **PELTON GH et al.** Altered PET functional brain responses in cognitively intact elderly persons at risk for Alzheimer disease (carriers of the ε4 allele). *Am J Geriatr Psychiatry*, 2004, vol. 12, 596-605
- **MOSCONI L** ; **NACMIAS B** ; **SORBI S** ; **DE CRISTOFARO MTR** ; **FAYAZZ M** ; **TEDDE A et al.** Brain metabolic decreases related to the dose of the APOE e4 allele in Alzheimer's disease. *J Neurol Neurosurg Psychiatry*, 2004, vol. 75, 370-376
- **KNOPMAN DS** ; **JACK CR JR** ; **WISTE HJ** ; **LUNDT ES** ; **WEIGAND SD** ; **VEMURI P et al.** 18F-fluorodeoxyglucose positron emission tomography, aging, and apolipoprotein E genotype in cognitively normal persons. *Neurobiol Aging*, 2014, vol. 35, 2096-106
- **PARANJPE M** ; **CHEN X** ; **LIU M** ; **PARANJPE I** ; **LEAL JP** ; **WANGET R et al.** The effect of APOE ε4 on longitudinal brain region-specific glucose metabolism in patients with mild cognitive impairment: a FDG-PET study. *Neuroimage Clin*, 2019, vol. 22, 101795
- Cummings J. Clinical and Biomarker Updates from BAN2401 Study 201 in Early AD. *Symposium at Clinical Trials in Alzheimer's Disease*, October 2018